# EUROPEAN PATENT APPLICATION

(11) **EP 2 805 962 A1**
(43) Date of publication of application: **26.11.2014**
(21) Application number: 11872934.2
(22) Date of filing: 04.10.2011
(51) Int. Cl.: C07K 5/06, A61K 38/08, A61P 37/04

(54) **IMMUNOMODULATOR METALLOPEPTIDES (IMMPS) AND COMPOSITIONS CONTAINING SAME**

(30) Priority: 28.09.2011 MX 2011010190
(71) Applicant: Universidad Autónoma del Estado de Morelos, Morelos 62209 (MX)
(72) Inventor: REYES ESPARZA, Jorge Alberto, Morelos 62209 (MX); RODRIGUEZ FRAGOSO, Maria de Lourdes, Morelos 62209 (MX); BADILLO JIMENEZ, Ana Laura, Morelos 62209 (MX); TORRES AGUILAR, Lorena Julieta, Morelos 62209 (MX); MARTINEZ OSUNA, Lorenzo Ulises, Morelos 62209 (MX); MARTINEZ DE LOS RIOS CORSINO, Abril Arianna, Morelos 62209 (MX); ARJONA CANUL, Carlos Antonio, Morelos 62209 (MX); MORALES ROJAS, Hugo, Morelos 62209 (MX)
(74) Representative: Vogel, Andreas
(86) International application number: PCT/MX2011/000117
(87) International publication number: WO 2013/048226

(57) **Abstract**

The present invention relates to the field of human and veterinary medicine. The invention solves the problem associated with the lack of an agent for improving the immune response in immunosuppressed individuals, or with autoimmunity problems as well as infections; reducing the effects of the stress, combating tissue fibrosis; the need for anti-inflammatory agents; treatments for acute and chronic hepatitis, treatments against malignant processes and metastasis, as well as thrombocytopenia, both in humans and animals. The present invention comprises a molecular complex formed by a metallic ion and a peptide as novel compound, which is denominated as an immunomodulator metallopeptide hereafter abbreviated as IMMP. Claims also include the process for obtaining the complex, the use of the metallopeptide for producing a therapeutic or nutraceutic agent, and the use of that agent in humans and animals.

SEQUENCE LISTING

<110> Applicant: UNIVERSIDAD AUTÓNOMA DEL ESTADO DE MORELOS

<120> Title of Invention: Immunomodulators metallopeptides (IMMPS) and
        compositions containing the same

<130> File Reference:

<140> Current Application Number: EP 11 872 934.2
<141> Current Filing Date: 2011-10-04

<150> Prior Application Number: MX/a/2011/010190
<151> PriorFilingDate: 2011-09-28

<160> Number of SEQ ID NOs:  1

<170> Software: BiSSAP 1.0

<210> 1
<211> 8
<212> PRT
<213> Bos taurus
<220>
<221> Source
<223> /mol_type="protein"
      /organism="Bos taurus"

<400> 1
Leu Glu Asp Gly Pro Lys Phe Leu                                        8

<210> 2
<211> 7
<212> PRT
<213> artificial sequences
<220>
<221> Source
<223> /mol_type="protein"
      /note="This is a molecule analogous to sequence 1,
       resulting from the deletion of L1"
      /organism="artificial sequences"

<400> 2
Glu Asp Gly Pro Lys Phe Leu                                            7

<210> 3
<211> 6
<212> PRT
<213> artificial sequences
<220>
<221> Source
<223> /mol_type="protein"
      /note="This is a molecule analogous to sequence 1,
       resulting from the deletion of L1 and E"
      /organism="artificial sequences"

<400> 3
Asp Gly Pro Lys Phe Leu                                                    6

<210> 4
<211> 9
<212> PRT
<213> artificial sequences
<220>
<221> Source
<223> /mol_type="protein"
      /note="This is a molecule analogous to sequence 1,
       the result of the addition of a XAA"
      /organism="artificial sequences"

<400> 4
Xaa Leu Glu Asp Gly Pro Lys Phe Leu                                        9

<210> 5
<211> 10
<212> PRT
<213> artificial sequences
<220>
<221> Source
<223> /mol_type="protein"
      /note="This is a molecule analogous to sequence 1,
       the result of the addition of two XAA before L1"
      /organism="artificial sequences"

<400> 5
Xaa Xaa Leu Glu Asp Gly Pro Lys Phe Leu                                   10

<210> 6
<211> 9
<212> PRT
<213> artificial sequences
<220>
<221> Source
<223> /mol_type="protein"
      /note="This is a hypothetical molecule, analogous to the sequence 1,
       the result of the addition of an after XAA L8"
      /organism="artificial sequences"

<400> 6
Leu Glu Asp Gly Pro Lys Phe Leu Xaa                                        9

<210> 7
<211> 10
<212> PRT
<213> artificial sequences
<220>
<221> Source
<223> /mol_type="protein"
      /note="This is a hypothetical molecule, analogous to the sequence 1,
       the result of adding two after XAA L8"
      /organism="artificial sequences"

<400> 7
Leu Glu Asp Gly Pro Lys Phe Leu Xaa Xaa                                 10

<210> 8
<211> 10
<212> PRT
<213> artificial sequences
<220>
<221> Source
<223> /mol_type="protein"
      /note="This is a hypothetical molecule, analogous to the sequence 1,
       the result of the addition of XAA before L1 and after XAA L8"
      /organism="artificial sequences"

<400> 8
Xaa Leu Glu Asp Gly Pro Lys Phe Leu Xaa                                 10

## Description

### TECHNICAL FIELD OF THE INVENTION.

The present invention refers to complexes having an immunomodulator effect in humans and animals, particularly to metallopeptides complexes capable of stimulating the immune response, for instance the T lymphocytes production, cooperators (CD4 +) and/or cytotoxic (CD8 +), and which are useful as active ingredients for the treatment of several diseases where the immune system stimulation is important.

### BACKGROUND OF THE INVENTION

Inflammation is a physiological response from the body to any kind of injury in order to maintain and restore homeostasis. It can be said that the inflammation is the first step of the repairing and involves vascular aspects which lead to the extravasation of fluid, and cellular involving leukocyte migration to the injury site. The vascular and cellular response is measured by chemical factors from damaged cells, plasma or leukocytes and which are activated by the injury or the inflammatory process itself. The process is activated, maintained and self-limited, by the so-called chemical mediators of inflammation, and is regulated by cytokines and chemokines. These molecules regulate the balance between factors promoting inflammation and those ones which stop it. This has lead to the interleukins division in proinflammatory IL-1a, IL-2, IL-4, IL-6, IL-8, IL-1, IL-13, TNF-alpha, and INF-gamma and anti -inflammatory such as IL -10 and TGF-beta. The IL-1 and TNF are produced by activated macrophages, active T cells and other cells. The secretion thereof is stimulated by endotoxin, immunocomplexes, toxins, physical injury, and the inflammatory process itself. Acute inflammation tends to be auto solved, which involves neutralization of chemical mediators that lead to the restoration of normal vascular permeability, the leukocytary infiltration absence, elimination of tissue leukocytes and the elimination of the interstitial fluid excess.

Chronic inflammation is that in which for weeks or months it can be observed active inflammation signs, tissue destruction and repairing attempts. It is characterized by infiltration of activated macrophages, lymphocytes and plasmatic cells, tissue destruction, mainly caused by the inflammatory cells, repairing attempts by the substitution of the injured tissue by conjunctive tissue (fibrosis) and the presence of angiogenesis. The most common causes of chronic inflammation are persistent infections, prolonged exposure to endogenous or exogenous toxic agents and autoimmunity. In this event, it is considered that IL-1 and TNF-α act to perpetuate the activation process and keep inflammation, so that blockage thereof can interrupt the process.

Cytokine secretion provides signals between cells of the immune system, with which it is coordinated the inflammatory response. Cytokines are a molecules group of proteinaceous nature and have a low molecular weight, which are secreted by cells such as lymphocytes, monocytes/macrophages, mast cells, eosinophils, among others. There are four main types of cytokine: interferon (IFN-α, IFN-β, IFN-γ), colony stimulating factors (G-CSF, M-CSF, GM-CSF), tumor necrosis factor (TNF-α, TNF-β) and interleukins (IL-1, IL-2, IL-4, etc.). Cytokines may have an autocrine action (over the cell same from which is secreted), paracrine (on the tissue where it is secreted) and endocrine (when is carried by the blood toward a distant cell on which it is acted. Apparently the main role of the cytokines is performed into a paracrine level (Eklund CM, 2009).

A defect in the complex process of controlling the expression and production of cytokine has been detected in several diseases as one of the involved pathophysiological mechanisms. Particularly a regulation loss of the proinflammatory cytokines secretion has been identified as involved in diseases such as asthma, psoriasis, septic shock, periodontal disease, rheumatoid arthritis, inflammatory bowel disease, inflammation presence after viral infection, eczema, autoimmune diseases, and neurodegenerative diseases (Desai D, 2009).

The presence and persistence of chronic inflammation is one of the main factors in the pathogenesis and autoimmune systemic diseases progression such as rheumatoid arthritis (RA) and systemic erythematosus lupus (SEL).

The immune response is strongly regulated from its beginning to its end by a set of peptide molecules generically called cytokines. Most of them have a very short half-life and are active at very low concentrations, even lower than 10⁻¹² M (molar peak), can act locally or systemically, which play a main role in the amplification and delimitation of the immune response. Thus, they play an important role in most of the pathological processes such as inflammation, infection, allergy, autoimmune diseases, cancer, etc.

The immune response is the mechanism that the nature and/or evolution have developed in order that top organisms defend themselves against any disease. Interaction between an antigen and the B or T cell receptors initiates a cascade of molecular and cellular events, called immune response, resulting in activation, proliferation and differentiation of these cells, leading to the antibodies production, cytokines, cells destruction and invaders agents and finally to the resolution and back to normal. When immune response fails the disease occurs. The failure may be due to an insufficient response (immunodeficiency) or an excessive response, little known, but occurs for example in cases of sepsis, with mass production of cytokines leading to shock and death; a third form of failure is "confusing" the appropriate with the non appropriate, which leads to self-injury or autoimmunity. That is why the modulation of the immune response plays an important role in the treatment of many diseases. Sometimes, it would be important to increase the immune response in patients with immunodeficiency, infection or cancer. On the contrary, it could be beneficial to inhibit or reduce the immune response in patients presenting alterations characterized by a widespread or chronic inflammatory process.

In adults, lymphocytes or T cells arise in the thymus from prolymphocytes coming from bone marrow. When the prolymphocytes arrives to the chemical and physical microenvironment of the thymus acquire the particular features of a lymphocyte. Maturation occurs in a set of steps including the acquisition of the T cell receptor (TCR), CD4 and CD8 co-receptors, loss of one of these and differentiation toward cooperative cell (CD4 +) or cytotoxic (CD8 +). In this process, dendritic cells of the thymic which act as presenting cells and interleukins 3 and 7 (IL - 3 and IL -7) are involved. The lymphocytes generation by the thymus is prominent in the step that is near to the birth and decays strongly after puberty, but the thymus is capable of producing them during the whole life, even in the senescence. Actually, all of the factors involved in the T lymphocytes differentiation, or in the reactivation of the generation thereof in subsequent stages of puberty, are not known.

We could say that once the inflammatory process has limited and restricted the damage, corresponds to the immune response the removal of the abnormal external or internal agents causing the injury, acting as a barrier against external pathogens and the abnormal cells growth by exerting its primary function, which corresponds to discriminate between the proper and the non proper, and deleting the latter.

The immune response is generally classified as humoral and cellular, the first one is represented by the presence of immunoglobulins or antibodies produced by plasmatic cells, in which the B-lymphocytes are converted after being activated. Antibodies inactivate pathogens through agglutination, precipitation, complement activation, and activation of the lysis depending from antibodies, among other mechanisms. The cellular response is represented by T lymphocytes, which are divided into cooperatives and cytotoxic or suppressors. Cooperatives act through cytokines and interleukins, regulating immune the immune responses by activating T and B lymphocytes, dendritic cells, macrophages, among others. Cytotoxic are particularly important in the response to viral infections and cancer, since they are able to destroy the infected or maligned cells inducing apoptosis or lysing them by perforins secretion.

In general, antibodies reach their maximum blood level, 2 weeks after the antigen challenge the first time that this occurs, and one week later on subsequent challenges due to the presence of memory cells. This time is a "helplessness" stage for the host, during which the infection, for example, may grow or spread.

Approximately 20% of the population suffers any autoimmune or chronic inflammatory disease, which is mediated by an abnormal immune response. An important feature of the autoimmune disease is the selectivity of target, a single type of cell, organ or tissue is target of the autoreactive cells populations. This is because only those cells have the activator antigen. An example of this includes diseases such as rheumatoid arthritis, diabetes mellitus type I, systemic erythematosus lupus and autoimmune hepatitis, all of which are characterized by chronic inflammation, tissue destruction and malfunction of the target organ. Autoimmunity can be defined as the loss of the tolerance to the proper. It can be originated from genetic alterations or environmental stress that exceeds the protection system against attacks to the proper (Christen U, 2004).

Although the therapeutic immune regulation concept focused to the treatment of autoimmune pathologies has been validated in several animal models, the development of strategies for the treatment of alterations of the autoimmunity in humans is still incipient. The main obstacle for this includes the contrasting results obtained from different models, as well as the contrasting functions of the regulatory T cells and the cytokines involved in the development of such disorders.

It is important to remember that the immune system is both the subject, and the object of regulatory control, and that generally the concept of immune regulation is predominantly used in specific reference to the elimination of external pathogens agents or to the protective immunity increase. It is certain that the regulatory mechanism is only one, very intricate and complex, but only one, constituted of multiple controls participating in the response and that when an imbalance arises, a system failure occurs, leading to a chronic infection, the apparition of a tumor, or a hypersensitivity reaction or autoimmune.

Autoimmune diseases are generally classified in the clinic for the presence of antibodies with affinity to particular antigens, such as Graves's disease having antibodies against the TSH receptor, or the presence of a cellular response, which mainly destroys cells and different tissues to those of the immune system also due to a response against particular antigens, such as Hashimoto's thyroiditis or diabetes type I. However, in many diseases there is a combination of both phenomena. Thus, Hashimoto's disease and diabetes Type I present autoreactive cells, but also the presence of autoantibodies. On the other hand, autoimmune diseases frequently study with an inflammatory component, including the expression of adhesion molecules on the endothelium, resulting in a defective leukocyte adhesion (Jimenez SA, 2004).

Therapies for chronic inflammation of autoimmune origins have been focused to the treatment of symptoms. The immunosuppressive agents used for treating autoimmune inflammation are focused to the response of the cells of the immune system or the cytokines produced by them. Are only partially effective for inducing remission (methimazole in Graves' disease), toxic (cyclosporine for diabetes type I), or simply palliative (corticosteroids for colitis or systemic erythematosus lupus) (Michelsen KS, 2004, Pasterkamp G, 2004; OudeNijhuis MM, 2007) between the new proposals of therapies for inflammation, rheumatoid arthritis and lupus, there are some which modulate the immune response (Sitkovsky M., 2007, Zimmerman DH, 2009,. B. White, 2010).

In some other events such as stress, cancer and fibrosis it has been detected that the modulation of the immune system is really important.

### Stress.

Stress is now recognized as the main "debilitating" or predisposing factor to various diseases such as cardiovascular, cancer, and those originated in a dysfunction of the immune system. Stress causes a variety of physiological changes, which include changes in the immune response, hormone levels, production and secretion of digestive enzymes and of the digestive function. Before a stressful event, stress hormones, such as norepinephrine, epinephrine and cortisol are released into the circulation to prepare the body for the fight-or-flight response. Stress in mammals is usually manifested with an increase in the body temperature due to changes in hemodynamics, increased heart rate and blood pressure. In patients with hypertension, the hemodynamic effects of stress can be particularly harmful, since they tend to aggravate hypertension, which is the main risk factor for cardiovascular disease.

It has been produced drugs that are effective in the control and treatment of stress and its consequences. Unfortunately most of them only fight the symptoms but not the pathophysiological process itself.

### Cancer.

Cancer is an uncontrolled growth of the progeny of a transformed cell, usually by accumulation of mutations or by an oncogenic virus. As a result of the malignization, one or more proteins produced by the cell will be mutated and when presented in the Type I Histocompatibility Main Complex (MHC I), it will be recognized as non-self, triggering an immune response and becoming eliminated by cytotoxic T lymphocytes or the natural killer cells (Vaux D, 1993). Thus the cancer clones that we can generate every day are eliminated by the immune system. When a failure of the immune system or immunosuppression takes place, malignant cells can survive, multiply and invade adjacent tissues. It has been demonstrated that the effector mechanisms of cellular and humoral immunity destroy *in vitro* and *in vivo* malignant tumor cells; in fact this constitutes the basis of some therapies that, for example use antibodies against the tumor cells, however, they have limitations.

CD8 cytolytic T lymphocytes (CTL) are in charge of the main mechanism of tumor immunity. These lymphocytes perform a monitoring function by recognizing and destroying cells that in their MHC I show protein antigens that are recognized as non-self. CD4 cooperative T cells (Th), particularly those of type I (Th1) also participate in the antitumor response, providing signals through cytokines, such as IL-2 which increases the efficiency of CTL. The Th1 cells also secrete tumor necrosis factor (TNF) and gamma interferon (IFN-gamma), which causes the tumor cells to increase expression of MHC I, and thus the posibilities of being recognized and eliminated by the CTL's. Also, the IFN-gamma, activates macrophages against tumor cells (Pasqualini D., 1996, J Maher, 2004).

The humoral immune response by antibody production also participates in the battle against the tumor, "marking" the tumor cells in order to be destroyed by the complement or by the macrophages. Also, antibodies can activate cytolisis mediated by antibody dependent cells (Benjamini E, 1996, Janeway C, 2001).

Tumor cells in turn, in their struggle to survive, develop mechanisms to evade the immune response. These include the decrease of the class I molecules expression, they quit to express antigens that are recognized by the TCR and thus, they could activate the cellular response; they may also hide these molecules under a glycocalyx, so that the complex class I cannot be able to interact with the T cells receptor and CD8 coreceptor of the cytolytic T lymphocytes, they can also secrete immunosuppressive agents, such as the beta transforming growth factor (TNF-β) or the expression of the Fas ligand and thereby inducing death in the immune cells expressing the Fas receptor, they can also produce soluble receptors for some cytokines such as IL-2, thereby impeding that CD4 cells to activate other cells of the immune system (Soddu S, 1992; Cefai D, 2001; Dworacki G, 2001 G. Pawelec, 2004; Ahmadzadeh M, 2005).

Metastases are the appearance of a secondary tumor at a site distant from the primary tumor. It constitutes the main cause of morbidity and mortality in cancer patients. Metastasis is due to a complex sequence of events that begins with the invasion of the adjacent tissues, intravasation and transportation through the lymphatic or circulatory systems, extravasation attachment to a secondary site, and growth in a tissue or a secondary organ. The molecular basis of tumorigenesis can vary greatly between one tumor and another, while the basic steps required for the metastasis are similar in all tumors. When a cell or a small group of cells are migrating, it is more susceptible to be attacked by the immune system, than those that are "joined" to the tumor.

Accordingly, therapies that can block metastasis could provide a powerful tool for the treatment of cancers with different origin. Thus, the development of this type of treatment should be the crucial therapeutic strategy for this problem.

Since the ideal way to cure cancer would be to destroy all tumor cells without affecting normal cells, one way to achieve this would be to induce a specific immune response against tumor cells.

Cancer is a serious public health problem, in Mexico is the second cause of death, while in the United States one in four deaths is due to cancer. The therapy against cancer is surgery, with which the tumor and the tumor cells are removed; however in many cases the complete removal is not possible due to its anatomical location, the invasion degree or metastases that is present. In such cases, chemotherapy is frequently used, for which drugs that kill tumor cells are used, however it is common to have the presence of resistant clones to said drugs that survive and repopulate the tumor generating distance metastases, thereby presenting tumor recurrence and death. In other cases radiotherapy is used with similar results to those of chemotherapy.

In order to achieve better therapeutic results, there has been many improvements, but still there is a wait for: effective therapies in the early stages of cancer, to reduce the amount of escapes, alternative therapies for the treatment of refractory tumors to current standard therapies, therapies for the reduction or elimination of metastases, with drugs less toxic, and better liberating systems of the drug.

The presence of antigens capable of activating the immune response in human cancers has been demonstrated, and that, when this response occurs, it may lead to tumor regression. Those antigens activate both types of immune response, humoral and cellular, whereby it can be assumed that they present epitopes capable of activating one of the different types of response (Coulie PG, 1997, Sahin U, 1997, Van den Eynde BJ, 1997; Wang RF., 1999). Based on these findings, it has become an objective to generate an immune therapy capable of inducing tumor regression. Although favorable results had been obtained, these have been sporadic and generally smaller in magnitude and frequency. These range from the development of vaccines using bacterial or viral tumor antigens, cytokines, and the use of antitumor antibodies, anti-cytokine or its receptors (Grange JM, 2008; Misaouel P, 2009; Trimble CL, 2009).

A problem in the effort to generate immune responses that produce tumor regression is that in cancer patients, these are generally found in a state of immunosuppression induced by the tumor as well as by the altered immune system of the patient itself (Yang L, 2004, Perez-Diez A, 2007), which has made non functional and impossible the use of immunizations consistently. Immune suppression or depletion reduces the ability of the immune system to respond. Such suppression can be induced by a drug or pathology such as AIDS induced by the HIV virus, or induced by a cancerous tumor, which shuts down the immune system (Tran H, 2008, Holtan SG, 2009).

A variety of immunization against tumor has been developed. However, these strategies are complex and significantly different from conventional immunization for other types of infectious diseases such as viral or bacterial ones (J Weber, 2000). One of such immunization strategies involves an antigen constituted by a Sialyl TN polysaccharide mucin conjugated with hemocyanin and administered with an adjuvant of Mycobacteria and a low dose of cyclophosphamide (MacLean GD, 1996). With this therapy, a good immune response has been obtained in a small number of patients with breast and ovary cancer. A large response means a tumoral reduction of more than 50% (Kreitman RJ, 2009, Mathew M, 2009; Shumway NM, 2009).

Gene therapy has also been attempted using adenovirus-based constructions, as a vector for the 16 peptide expression of the papilloma virus (HPV) for the vaccination of patients with cervical cancer and it has provided a satisfactory answer in a low percentage of patients (LK Borysiewicz, 1996).

Dendritic cell therapy has also been attempted. Dendritic cells were previously exposed to peptide fragments of the prostate specific antigen in patients with metastatic prostate cancer, obtaining a good response in a low percentage of patients (Sanda MG, 1999).

Additionally, autologous tumors have been used with low doses of cyclophosphamide and BCG to immunize patients with melanoma. But few clinical answers have been obtained (MF Mastrangelo, 1996; Berinstein NL, 2009). Another strategy consists in the use of antigens MAGE with a variety of adjuvants. This has also reported poor results in patients.

In the last years there have been several patent applications, whose main objective has been focused on generating a treatment for cancer with different approaches, for example to block or inhibit cytokines (Jassoy C, 2010; Miossec P, 2010, Nishimoto N, 2010), eliminate cytokine soluble receptors, for IL-2 (H. Sicard, 2010), for IL-6 in mesothelioma (Hadashi, Y, 2010), which increase the immune response against the tumor (Hadeen JW, 2009, Vollmer J, 2009), application of interleukins such as IL-20 for the treatment of cervical cancer (Chandrasekher YA , 2010), and for the treatment of metastases.

Today, treatment with IL-2 has shown minor effects in two types of cancer renal, and melanoma (with a rate of responses r lesser than 20%). It has been considered ineffective in squamous cell cancer of head and neck and in prostate cancer (McDermott DF, 2009, Shaker MA, 2009).

It is important to contrast the prevention with "classical" antigens of complex structure and high molecular weight in healthy patients versus treatment (generaly failed) with tumoral or peptide antigens in immunosuppressed patients. The first is easy and it is represented by viral vaccines, which attest to its effectiveness. The latter, after 30 years of failed attempts have proved being almost impossible.

In several of the above strategies the attempt to increase or generate an immune response against the tumor has been tried, whether humoral, cellular, or both. However, the success has been limited and inconsistent. Therefore it is necessary to develop a method capable of removing the patient's immunosuppression, anergy against tumor antigens and/or able to defeat the mechanisms of tumor evasion.

### Liver damage induced by toxic agents and drugs.

It is considered that the liver damage induced by drugs may be through two general mechanisms, one intrinsic to the drug and other idiosyncratic. The intrinsic damage is due to a particular characteristic of the molecule that produces hepatocellular injury in a predictible manner and dependent, on the dosage, this damage can be direct by the drug or by one of its metabolites. The idiosyncratic damage is the type of damage most frequently produced by drugs and can it can be divided into metabolic and immune; producing an allergy or hypersensitivity to the drug. The intrinsic damage is characterized, in general, by cellular necrosis with little inflammation, while idiosyncratic reactions to drugs are often characterized by a secondary injury to the inflammation.

Liver injuries can also be divided into hepatocellular, cholestatic or mixed based on the criteria of the Council of International Organizations of Medical Sciences (PB Watkins, 2006; Teschke R, 2008).

10 % of acute hepatitis in the United States is due to liver damage induced by drugs, however the most common pathology is caused by them is cholestatic hepatitis. Fulminant hepatitis is characterized by the occurrence of hepatic encephalopathy within a period of 8 weeks after the hepatitis symptoms apparition. This is the type of damage that occurs most frequently in the USA, being from 25 to 50 % of them (1996 R. Williams, R. Williams, 2003; Hanje AJ, 2007).

Chronic hepatitis refers to persistent biochemical alterations beyond 6 months, although in some studies a time of 3 months is considered for hepatocellular injury and 6 months for the cholestatic or mixed. From 5 to 10% of the cases of drug-induced hepatitis evolve to chronicity. This proportion is higher in the drugs that produce cholestasis or a mixed pattern (Be'nichou C. , 1990, Batts KP, 1995).

Many drugs can cause chronic hepatitis which is serologically and morphologically indistinguishable from de novo autoimmune hepatitis. Liver disease can be accompanied by hyrsensitivity data such as rash, arthralgia, and eosinophilia.

There are few effective drugs for the treatment of hepatitis. For those of viral etiology, particularly hepatitis C, peg-interferon and ribavirin are used, recentlyit has emerged a new type of interferon, "concensus", which has been useful in cases resistant to the peg-IFN (Hsu HH., 2008).

### Platelets.

Platelets are involved in hemostasis and wound repair. Thrombopoiesis is performed on the bone marrow; it is dependent from the microenvironment of this organ, composed of cells, extracellular matrix and growth soluble factors. Various growth factors are involved in the generation and maturation of the platelets; they include IL-3, the stimulating factor of granulocytes and macrophages colonies, IL-6, IL-11, the inhibitory factor of leukemia and the thrombopoietin, the latter two being indispensable. The liver is the organ that produces the greatest amount of thrombopoietin, which appears to be constitutively. In patients presenting liver disease, thrombocytopenia is frequently observed. In cases of inflammation the IL - 6 induces an increased production of thrombopoietin (Kaushansky K., 2009).

Generally, a negative regulation mechanism acts, thus, when there is a high number of platelets, a low level of thrombopoietin is observed, and vice versa. However, this does not always occur, for example in cases of excessive destruction of platelets it is possible to find normal levels of thrombopoietin, there is neither a correlation of the platelets levels and thrombopoietin in cases of inflammation of the endothelium (K. Kaushansky , 2005).

In patients with hepatitis it is common to observe thrombocytopenia, generally as a sign of an advanced disease. This has been attributed to an increased sequestration of platelets by the spleen, the alteration in the production of thrombopoietin or other growth factors produced by the liver (Afdhal N, 2008, P Witters, 2008; Tillmann HL, 2010).

Treatment of thrombocytopenia depends on its etiology, but in general includes splenectomy, corticosteroids, danazol, and recently, eltrombopag, a molecule that mimics the effects of thrombopoietin (HL Tillmann, 2010).

As mentioned above, the modulation of the immune response plays an important role in the treatment of many diseases. Sometimes it would be important to enhance the immune response in patients with an infection or cancer. On the contrary, it could be beneficial to inhibit or reduce the immune response in patients presenting disorders characterized by an inflammatory process.

Biological response modifiers (such as antibodies, immunotherapeutics, and mimetic peptides) are agents that modulate the defense mechanisms of living organisms or biological responses, such as survival, growth or cells differentiation to direct them to have an antitumor activity.

Adjuvants are compounds which when are administered to an individual or tested in vitro, increase the immune response to an antigen in a subject to which the antigen is administered, or increases the activity of certain cells or certain activities of immune system cells. A large number of compounds presenting this activity have been prepared and tested. However, most have toxic effects, nonspecific, are unstable or their effect is very low.

Efforts to treat autoimmune alterations have been directed to attack the immune system cells or their products using antigens, T cell peptides, monoclonal antibodies (mAbs), recombinant proteins acting as ligands for cellular receptors, TNF-alpha type molecules, or IgE, all of these being passive agents, their main problem is that they have to be administered to the patient for life, likewise, Mabs or soluble receptors should be "humanized" to prevent rejection and adverse events it entails. It is not convenient that the treatment eliminate whole populations of T lymphocytes or other cells from the immune system that are required for a correct immune response against pathogens. Therapy should only act on the main molecule or "abnormal" cell.

There are also active agents in the treatment of autoimmune diseases, their objective is to induce that the body produces molecules to counterattack or neutralize the agent. An example of active agent are vaccines that induce a long-term adaptive response through the production of antibodies or cytotoxic T lymphocytes.

Given the large number of diseases in which is involved the regulation loss of the proinflammatory cytokines levels, there is a need for new molecules and/or methods to treat pathologies which involve an increase in the proinflammatory cytokines.

To treat autoimmune diseases, it is necessary a strategy that requires greater specificity, lower dosages and less frequent administration of active substances. The therapeutic agent should have the option to be applied in various ways, likewise, it would be convenient that the molecules be under 3000-5000 Da so that they can cross the blood -brain barrier.

The therapeutic agent should induce a homeostatic immune response that is physiological, activating the cells that are needed in the necessary site. For example, increase the cooperative T lymphocyte subpopulations, Treg and/or the production of some cytokines such as IL-2 and by the contrary, decrease the production of others with proinflammatory function, such as IL- 1.

It is desirable that production of the therapeutic agent be highly reproducible in quantity and quality, not very elaborated or expensive and it is desirable that it is stable during its storage and easy to use.

The problems raised here have tried to be solved by using a variety of immunomodulators, just to mention some options: the production of costimulatory molecule antagonists, such as B7 (Zou W, 2008), antibodies and agents that block the interaction of CD-40 and its ligand, therefore they modulate the survival of the cells expressing it, modifying their proliferation (Luqman M, 2008), adjuvants such as RC-529 (one alkylamino glucosaminide) and QS-21 (a saponin), administered jointly with the antigen (vaccine) they have been used to increase the immune response in mammals, in vaccines against: cancer, viral, bacterial or protozoal antigens and against autoantigens involved in autoimmune diseases, some chemical agents have been used as triazol pyrimidine tricyclic derivatives (Isoda S, 1989), antigenic peptides (Smith III GJ, 2009), the use of phenyl methimazole or methimazole derivatives for the treatment of diseases with Toll-like receptor 3 and 4 (TLR3 and TLR4) overexpression, IkB kinase (IKK) inhibitors (Ginn JD, 2007); alkylamino carboxy cycle derivatives which are modulators of the esfingosine-1-phosphate (S1P) receptors (Bhattacharya SK, 2009), antagonist antibodies against some B cells receptors (Novobrant - Seva T., 2006). In the case of fibrosis, inhibitors of a lysyl oxidase (LOX) and/or molecules similar to it are being used.

### Immunoregulatory thymic.

The thymus is a lymphatic system organ, responsible for the maturation of T lymphocites, and endocrine since it secretes some hormones. The thymus has its maximum activity during the neonatal period and childhood. In adulthood it partially atrophies, being replaced by adipose tissue, yet it always retains a residual activity.

The thymus exerts a clear influence over the development and maturation of the lymphatic system and in the defensive immune response of our organism. The thymus is a primary lymphoid organ in which the differentiation of undifferentiated lymphosites (T lymphoblasts) that came out of the bone marrow takes place, they enter the thymus and go colonizing different areas thereof, while maturing and differentiating. The first colonized area is the superficial cortex. From here they pass to the deep cortex and finally to the thymic medulla. Throughout this journey, the T lymphoblasts acquire specific antigenic receptors (T cell receptor, CD3, CD4 and CD8 co-receptors, among others) and they learn to not attacking the individual's own antigens (autoantigens), becoming mature T lymphocytes.

The thymus can also be considered an endocrine system organ and therefore an endocrine gland, since it secretes hormones and other soluble factors, which besides controlling the production and maturation of T lymphocites in the thymus, regulate the activity and interactions of T cells in the peripheral tissues. Polypeptides secreted by this organ with hormonal characteristics are known, these are thymulin or serum thymic factor (STF), thymic humoral factor (THF) and thymopoietin or thymosin. From both these proteins as well as thymus extracts (of uncertain composition) called factors, properties for the treatment of immunodeficiencies in different degrees have been described.

The THF is an immunologically active soluble fraction described since 1975 (Kook et. al., 1975), the THF- gamma2 with the sequence: Leu-Glu-Asp-Gly-Pro-Lys-Phe-Leu (LEDGPKFL), is the active principle of the THF. The Patent EP0204328B1 (Trainin N, 1986) refers precisely to the THF peptide with the sequence Leu-Glu-Asp-Gly-Pro-Lys-Phe-Leu extracted from thymus glands and which is denominated THF gamma 2. The THF-gamma 2 participates in the differentiation of T cells and in their proliferation in the three compartments of the immune system: the bone marrow, the thymus and the peripheral lymphatic system, some of these functions have been observed only in vitro while others are manifested also in vivo, therefore its physiological role is not clear (Granoth, R. et. al., 1997).

The thymic serum factor, its formulations and its properties have been described by several authors: JF Bach et . al. enNature (1977 ) and in the Patents (DF Veber, 1978, Bach JF, 1979), the latter describes the possibility of using them as medicaments for autoimmune diseases, because of its immunoregulatory properties, it improves the immune response both cellular and humoral, which stimulates selectively the activity of T cells (Haddad , J. 2009).

In particular, the THF has shown to have various biological activities, such as increasing the proliferative response of T cells to mitogens, the reaction of the mixture of lymphocytes, and the production of IL-2 induced with concanavalin A. The latter at concentrations of 100 to 300 ng/ml (1.09 to 3.2 x 10-⁷ M), in umbilical cord blood, the THF (300-600 ng/mL) (3.2 to 6.4 x 10-⁷ M) also increases the production of IL -2-induced with PHA (Burstein 1988; Burstein, 1999). The administration of 4ng/Kg of THF twice at week is able to restore the immune response in thymectomized mice in neonatal stage. Its administration restores the immune response on mice infected with cytomegalovirus, an opportunistic virus. The administration of this factor, in amounts of 0.4 ng/doses at 50 µg/doses, prolonged the survival of mice bearers of a plasmacytoma and that received chemotherapy with 5-fluorouracil. Likewise, it also improved the immune response in mice with some types of tumor, such as plasmacytoma, that received chemotherapy or radiotherapy. Furthermore, it is capable of improving the decrease of the immune response due to aging. In bone marrow, the THF increases the production of granulocytes and macrophages, besides the erythroid line (ZT Handzel 1990; Ophir R, 1991, Barak Y, 1992, Burstein, 1998). In pilot clinical studies with patients presenting lymphoproliferative disorders, neuroblastoma and rhabdomyosarcoma THF was used for three weeks in daily injection at a dose of 4 ng/kg/day, as adjuvant to chemotherapy and maintenance therapy. The presence of signs of infection, fever, general condition and different blood parameters were monitored, observing that there was no infection or fever, but a good general condition, also it was observed an increase in the CD4/CD8 relation and of the NK cells. Likewise, in another pilot clinical study including patients with deficiencies in the immune system and severe herpes virus infections, with the scheme mentioned above, a regression of the viral infection and an increase in the T cell population was observed (Handzel ZT, 1990; Burstein, 1999).

The pre-incubation for one hour of normal bone marrow cells in the presence of THF, increases from 2 to 5 times the number of myeloid colonies in the presence of the stimulating factor of colonies of granulocytes and macrophages (GM -CSF), but not when incubated alone. The reported optimal concentration for this purpose is in the range of 25 to 100 ng/mL (27 x 10⁻⁹ to 10 x10⁻⁹ M) (M Pecht, 1993; Burstein, 1999).

Attempts have been made for improving the potency of the immunoregulatory properties of the THF by making certain modifications, for example, in the U.S. patent 5968898 (Burstein And Trainin N. et al, 1999) analogs of THF-gamma2 with the same sequence are described, but it also states deletions substitutions cyclizations of amino acids, bonding with other molecules, with which it is achieved an increase of a 30% in the production of IL-2 induced with concanavalin A in spleen cells, as well as an increase in the formation of progenitor cells of granulocytes and macrophages colonies in the bone marrow of mice (Barak et al 1992 and Petch et al 1993). Granoth et. al. report the activity of chimeras conformed by tufsin (a phagocyte stimulator peptide) and THF, tuftsin-THFgamma 2 chimeras and THFgamma2-tufsina, increased the response of the lymphatic cells *in vivo* in presence of antigen KLH in 40% and 80 %, respectively.

Due to the above, it is necessary to have immunomodulatory and immunostimulatory compositions of the immune system in animals, which allow treating the above conditions efficiently.

### BRIEF DESCRIPTION OF THE FIGURES

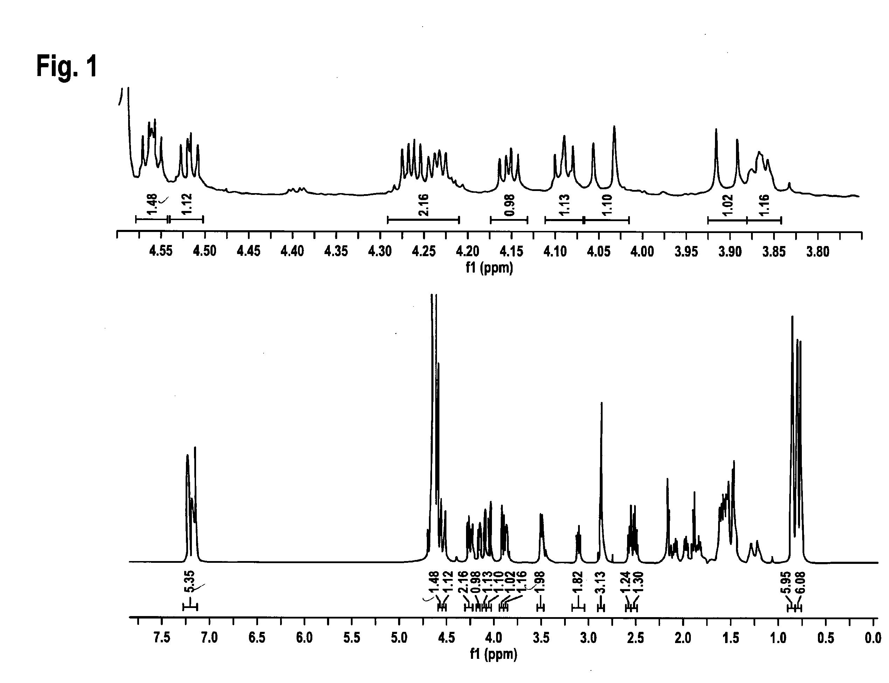

**Figure 1****.** Depicts the 1 H RMN spectrum (700 MHz) for the LEDGPKFL peptide in D2O. The upper part shows the expansion in the zone of the Hα pointing out the nine expected protons in the peptide.

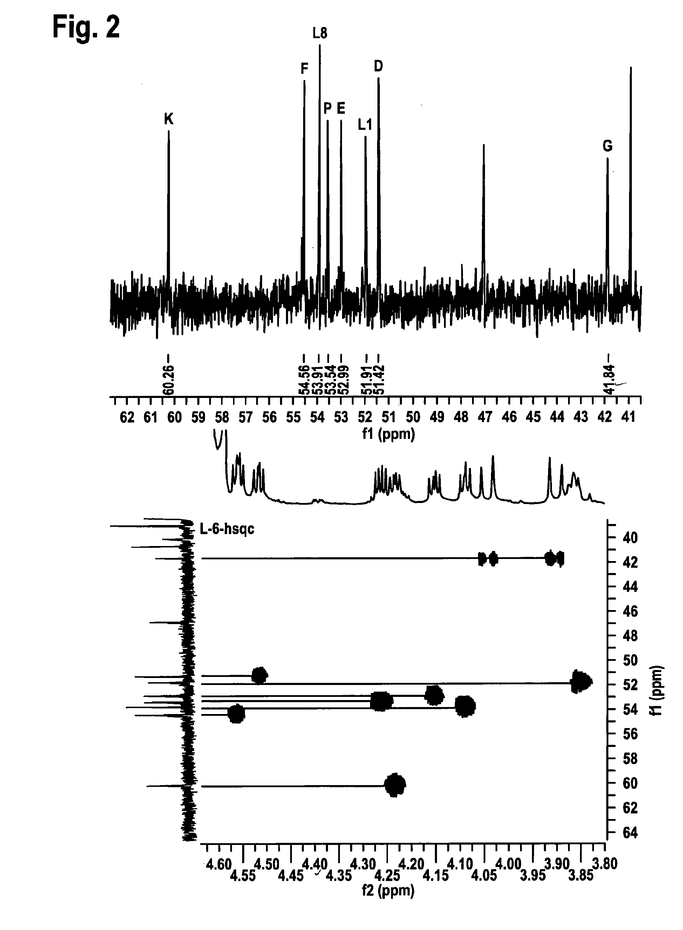

**Figure 2****.** Depicts the 13C RMN spectrum (176 MHz) for the LEDGPKFL peptide in D2O. The upper part shows the expansion in the zone of the Cα pointing out the eight expected carbons in the peptide. The lower part shows the correlation spectrum 13C-1H (gHSQC). It is shown Leucine of the position 1 (L1) as well as the Leucine of the position 8 (L8) of the peptide.

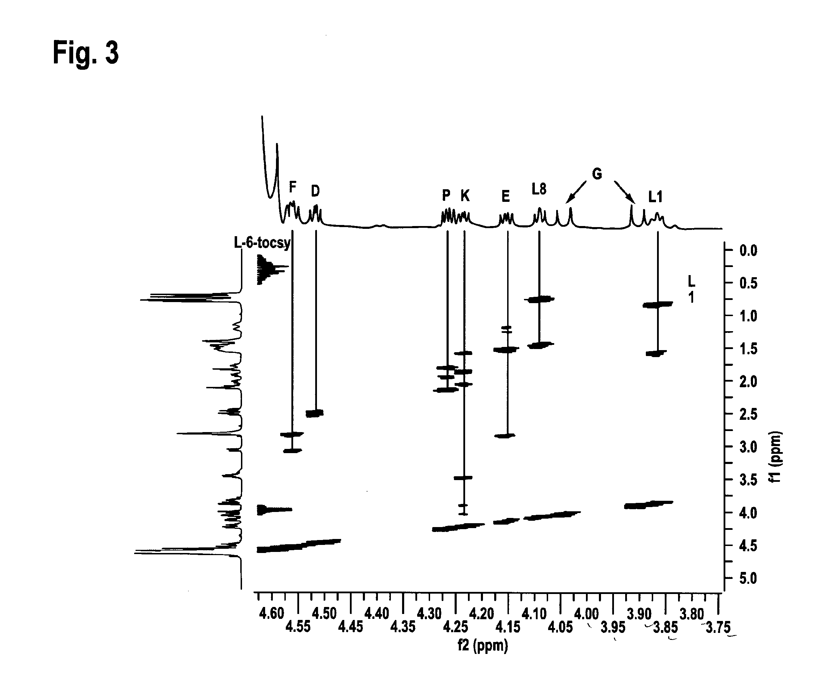

**Figure 3****.** Depicts the RMN 1 H spectrum (700 MHz) for the LEDGPKFL peptide in D2O. The upper part shows the expansion in the zone of the Hα pointing out the assignation of the eight expected aminoacids in the peptide. It is shown Leucine of the position 1 (L1) as well as the Leucine of the position 8 (L8) of the peptide.

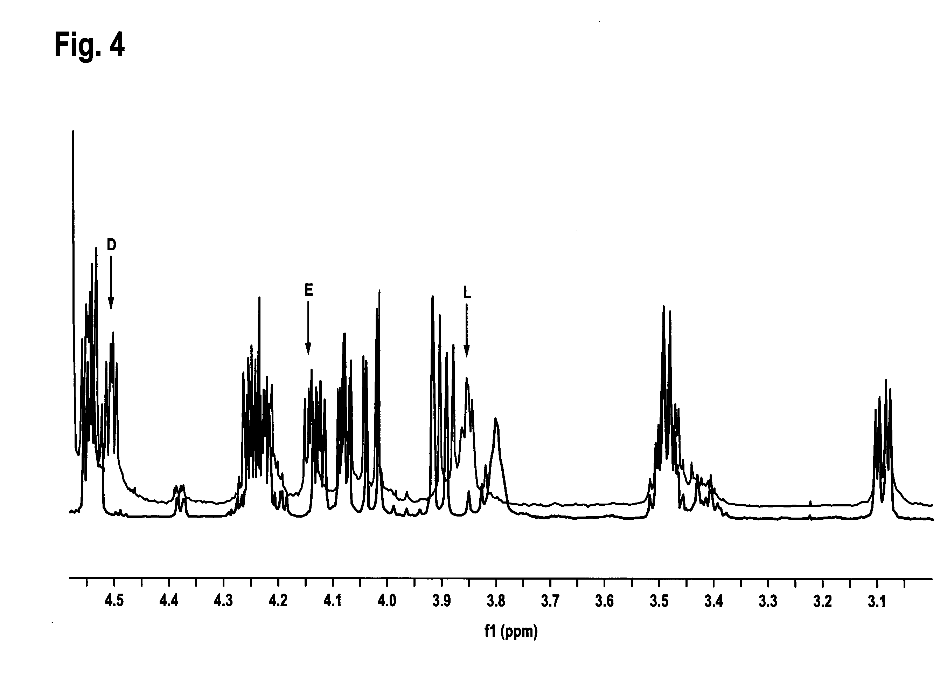

**Figure 4****.** Depicts the RMN 1 H spectrum (700 MHz) for the LEDGPKFL peptide in D2O. LEDGPKFL 15 mM (upper graphic) Peptide and LEDGPKFL 15 mM and ZnCl2 15 mM (lower graphic) peptide. It is shown the expansion the in the zone of the Hα pointing out the assignation of the aminoacids suffering a chemical displacement in presence of a ZnCl2 molar equivalent.

### DETAILED DESCRIPTION OF THE INVENTION

The present invention represents a technological breakthrough providing an agent capable of modulating the immune response and inflammation, which contributes to improve the immune response in humans and animals, and at the same time it covers the need of that with its use, there are no side effects or adverse effects for the animal organism, including the human organism.

The present invention refers to immunomodulators metallopeptides and to compositions containing them, wherein said IMMP are complexes formed between a divalent ion, preferably selected from the group consisting of Zn2+, Cu2+, Mg2+ o mixtures thereof, and a peptide of formula I,

Xn-Asp-Gly-Pro-Lys-Phe-Leu-Xm I

Where: Xn and Xm are any independent aminoacid one from another,
n has a value of 0 to 4,
m has a value of 0 to 2, and
wherein the peptide and the divalent ion are joined by non-covalent linking between each other, represented for example by the LEDGPKFL (SEQ. ID. 1) sequence, their analogues and variations, including SEQ. ID. 2 to SEQ. ID. 10 and variants of any of said described sequences to which the amino and terminal carboxy ends had been blocked for being considered variants of the original peptide. It is also included, under this term, in the context of this document: analog, derivatives, conjugated, metabolites and precursors of the peptide.

Another embodiment of the invention refers to the IMMP use and analog peptides, in the dose, posology, pathways and forms of suitable administration.

Besides the complexes metallopeptides which are described in detail below and which correspond to analog actives, are located into the embodiments of the invention, the nucleotide sequences encoding them, vectors or plasmids containing said nucleotide sequences, the process for obtaining the zinc-peptide complexes, the use of the complexes for medicaments manufacturing including nutraceutical and in order to treat with them the conditions in which are required induction in the production of T lymphocytes, (CD4+) and/or cytotoxic (CD8+), in humans and generally in animals, improving the immune response in patients with secondary immunosuppression; modifying the immune response and accelerating the antibodies production against an antigen or vaccine in humans and animals; acting prophylactically to protect subjects at risk, against an infectious disease and its effects, eliminating, combating or reducing the effects of an acute infection, chronic infection and recurrent infection; eliminating, combating or reducing the effects of an autoimmune disease or hypersensitivity; eliminating, combating or reducing the effects of stress; preventing, eliminating, combating or reducing the effects of fibrosis; eliminating, combating or reducing the effects of cancer; preventing, eliminating, combating or reducing the effects of metastases; preventing, eliminating, combating or reducing the effects of acute or chronic inflammation; preventing, eliminating, combating or reducing the effects of acute or chronic hepatitis, infectious, toxic or autoimmune; preventing, eliminating, combating or reducing the effects of liver cirrhosis; preventing, eliminating, combating or reducing the effects of rheumatoid arthritis; preventing, eliminating, combating or reducing the thrombocytopenia derease caused by liver problems o the other type.

Within the scope of the invention regarding the IMMP use of the present invention, it is considered that the individual to which it can be applied the medicament containing the immunomodulator of the invention (IMMP) as active component, can be either in a healt condition, in which case the utility of the agent is to prevent disease, or could be pursuing any disease or imbalance such as any of those mentioned in the preceding paragraph or another.

Are also embodiments of the invention any pharmaceutical form containing the IMMP complex of the invention as active or therapeutic agent, for example, injectable pharmaceutical forms, tablets, capsules, oral solutions or suspensions, local or general application solutions, powder to be dissolved, nutraceutical compositions or those forms which are convenient and compatible with the IMMP complexes of the present invention. They can be in the form of single or multiple dosages, in both cases in suitable discrete physical units for use in the different animal species. Each unit contains a specific and adequate quantity of the active and/or diluent ingredient, as well as carriers, as needed.

The terms "complex", "metallopeptide complex" and "IMMP" are used indistinctly in the present application to refer to the complexes formed by a divalent ion and the formula I peptide, their analog and/or derivatives, as well as the material obtained by the method of the present invention.

Are also embodiments of the invention prophylactic or therapeutic methods concomitant to the use of IMMP complex, each one addressing different conditions of the human or animal requiring the biological activity of the IMMP.

During development of the present invention, it was investigated in the prior art regarding activities of the LEDGPKFL (THF) peptide, wherein it was found that in the in vitro studies their biological effects were not constant, and that in vivo the reports of its use efficacy were not conclusive, since other authors agree with this lack of specificity about the physiological role of THF from inconsistencies between the results with in vivo and in vitro experiments.

As shown below in the examples, the LEDGPKFL (THF) peptide has a much smaller effect and just to higher concentrations in two magnitude orders over the proliferation and differentiation of rat thymocytes, main effect attributed to this factor. Based on our results (Tables 1, 2, 3, 5, 6, 10, 11, 12 and 13), we noticed that LEDGPKFL peptide only exerted effect on monocytes, thymocytes and human lymphocytes or those from animal origins, if these were co-estimulated with lipopolysaccharide (LPS), concanavalin A or phytohemagglutinin A respectively, thus, we concluded that the IMMP acts on "activated" thymocytes and T cells, and not on those " at resting" or " inactive", which is particularly important.

Thymus is the organ with the highest amount of zinc in the organism, the thymic hormone called thymulin, in its joined form to the zinc, is important for the T cells maturation. Zinc is also required by metalloproteinases, in fact these have a higher affinity for zinc than thymulin, so that in case of zinc deficiency, the thymus efficiency is reduced, a reason for this could be precisely to the low quantity of zinc that is available for the thymulin, founding that such a deficiency occurs with the age (Mocchegiani, E. 1998). Thymulin sequence and the description of its affinity for zinc, at pH 7.5 with a apparent affinity constant of Kdof 5±2·10-7 M, wherein this union is pH dependent, thymulin union does not occur at a pH below of 6.0 and the Ga3+, AI3+2+ y Cu2+ can compete with the union at Zn2+ to one of pH 7.5. The activity which denotes said union, is also described by Gastinel, LN et. al. (1984).

During development of the present invention it was investigated the role of zinc ion in the stimulatory activity of the cellular proliferation of THP-1 cells (human monocytes) presenting the LEDGPKFL peptide; the tests showed (Table 1) that the peptide alone at concentrations of 10-8 M (9.18 ng/mL) stimulates the cell proliferation, said concentration is close to that reported in the literature for the peptide to other cells (23 ng/mL) (2.5 x 10-8 M). But when the LEDGPKFL peptide is added with the zinc ion, it was observed the same activity but completely out of the observed parameters and in an unexpected manner at a lower concentration of more than 200 times (0.0983 ng/mL o 1.0 x 10-10 M). That is to say, the zinc ion presence increased the potency of the peptide more than 200 times. The previous test was repeated with human lymphocytes (Table 2), newborn rat thymocytes (Table 3), and monolithic cells (Table 4), wherein it was observed again the higher effect in the presence of the peptide and zinc ion. Like in the above described test, the potency of the zinc and peptide mixture is more than 200 times higher than that of the peptide alone. On the other hand, it was observed that the zinc by itself induces poorly the proliferation of cells, so that the observed increase in the potency is not due to the activity of the zinc ion by itself.

The zinc used to form the metallopeptide complexes of the invention can be from any molecule able to donate it, usually a chloride or sulfate.

To identify if zinc is an essential component for the activity of the peptide or if it is a synergistic effect of the zinc on the peptide activity, some tests were performed in presence of diethylene-triamine-paraacetate (DTPA), an agent that captures the zinc from the extracellular medium, wherein it was found that the peptide lost all biological activity (see tables 4, 5, 6 and 7), ie, it did not stimulate the proliferation of THP-1 cells, Jurkat or human lymphocytes.

To determine if the peptide conforms a molecular complex with the zinc ion, it was performed studies of nuclear magnetic resonance (NMR), so as to see if the spatial configuration of the peptide is modified in the presence of zinc ion. When the NMR of proton and carbon was performed it was observed effectively, the spatial configuration of the peptide is modified in the presence of zinc. Particularly the amino acids that have a carboxylic acid present a chemical desplacement in the presence of zinc ions. Glutamic acid (D) changes towards downfield (Δδ +0.027 ppm), aspartic acid (E) and the leucine with the L terminal carboxylic acid suffer a displacement towards highfield (Δδ -0.016 and - 0.051 ppm, respectively). These displacements show that the LEDGPKFL peptide is only molecule prior state to the biologically active (precursor) that is characteristic of the molecular complex zinc-peptide, capable of acting on activated cells of the immune system and mediators of the inflammation.

To investigate whether another physicochemical feature of the peptide is modified by performing the complex of the present invention, it was performed a high resolution chromatography in inversed phase, in which the polar molecules are firstly eluted, and subsequently those which are the less polar, we observed that the retention time of the peptide and zinc-peptide complex were different in 230 milliseconds, which indicates that the ion neutralized some charges present in the ion (see table 9).

In order to investigate whether the peptide alone could form molecular complexes with the zinc ion or could do so with other ions, tests under the same conditions, with Mg2+, Cu2+, y Mn2+, were performed, wherein it was observed that the first two ions were able to increase the potency of the peptide, but not the manganese ion. However, the potency of the complex formed with Mg2+ y Cu2+ ions is 10 times lower (Table 8). By making retention studies in a column of high resolution chromatography, using an inverse phase technique, it was observed that the addition of Mg2+ y Cu2+, it was also able to decrease the retention time, but for less time than the Zn2+. Therefore, we conclude that the peptide can make metallopeptide complexes with zinc ions, magnesium and copper.

The present invention refers to metallopeptide complexes of formula I, for example THF complexes joined to zinc or other divalent cation, called IMMP, this complex having an immunomodulatory and anti-inflammatory activity not previously described, being able to modulate the cytokines levels, both locally and systemically, thus, it is useful to modify different aspects of the immune response both cellular and humoral. According to the previous, the complexes of the present invention are able to alter the development of different kinds of diseases such as infections, autoimmune, acute and chronic inflammation and cancer, besides increasing the response to vaccines or immunizations, improving the humoral and cellular response, including levels of activated T lymphocytes and generating an adequate immune memory, thus, it has great potential for medical use in humans and animals. The zinc effect for immunoenhancing the effect of vaccines in chickens, by means of thymichastimuline and thymopoietin (including an active peptide thymopoietin called thymopentin) hormones, including only the zinc salt, is reported by Barbour, et. al. 1998, finding differences in patterns and levels of immunoenhancing.

The IMMP of the invention is an immunomodulator metallopeptide complex that can be used for treating a human or animal subject to modify its immune response, modifying the inflammation, to threat preventive, therapeutic or palliatively an infectious disease, physical or psychological stress, cancer, metastasis, autoimmune disease, fibrosis, thrombocytopenia, or any other in which it can exert a positive effect.

The IMMP is a metallopeptide molecular complex, in which the peptide has the aminoacids sequence represented by formula I, analogue peptides or derivatives of modifications thereof, while the metal ion may be selected from the group comprising divalent ions of zinc, magnesium, copper, or combinations thereof, and having an modifier effect of the immune response and inflammation, capable of promoting the health and growth of the subjects who receive it.

The molecular weight of the molecular complex IMMP, when is conformed for example, by the peptide having the SEQ. ID. 1 and the metal ion zinc2+, is 983.4 g/mol, however, said weight can vary according to the peptide and the metal ion used for the formation of the complex of the invention.

The peptide participating in the metallopeptide complex of the invention and represented by the formula I, can be obtained by different methods: isolated from animal tissues and organs when the peptide is that of the SEQ. ID. No. 1, or chemically synthesized, by any of the known techniques or those that will be known, by DNA recombinant technology, or biotechnology, and even in an endogenous manner stimulating its production. For their use in vitro and/or in vivo, the peptide involved in metallopeptide complex of the IMMP, its analog or derivatives thereof can be chemically modified to prevent being degraded by peptidases and increasing its lifetime in an organism, for example by chemical protection through amidation or acetylation of the terminal groups, among others.

Therefore, the complexes of the present invention are a product to improve the immune response and inflammation, and contribute to get better vaccines enhancing their effect; improving the immune response in normal subjects, immunosuppressed or with hypersensitivity or autoimmunity disorders; stimulating immune response in patients with acute, chronic or recurrent infections; reducing the effects of stress, both physical and psychological; combating tissue fibrosis, for instance during the development of liver cirrhosis, the need or not for new steroidal anti-inflammatory agents, which are also safe when are used to combat acute and chronic inflammation; the need for new treatments which are effective and safe for acute and chronic hepatitis, infectious, toxic or autoimmune; increasing the immune response against malignant tumoral processes and metastasis, all of that in humans and animals.

In one of its preferred embodiments, in the IMMP complex the peptide of formula I can have the following sequences:
Leu-Glu-Asp-Gly-Pro-Lys-Phe-Leu (SEQ. ID. No. 1),
Glu-Asp-Gly-Pro-Lys-Phe-Leu (SEQ. ID. No. 2),
Asp-Gly-Pro-Lys-Phe-Leu (SEQ. ID. No. 3),
X-Leu-Glu-Asp-Gly-Pro-Lys-Phe-Leu (SEQ. ID. No. 4),
X-X- Leu-Glu-Asp-Gly-Pro-Lys-Phe-Leu (SEQ. ID. No. 5),
Leu-Glu-Asp-Gly-Pro-Lys-Phe-Leu-X (SEQ. ID. No. 6),
Leu-Glu-Asp-Gly-Pro-Lys-Phe-Leu-X-X (SEQ. ID. No. 7),
X-Leu-Glu-Asp-Gly-Pro-Lys-Phe-Leu-X (SEQ. ID. No. 8).

In another embodiment of the present invention, the molecular complex of the invention comprises a metallopeptide complex, wherein the peptide is precursor or derivative from the peptide of formula I, as well as the analog that are obtained therefrom by deleting one or two contiguous amino acids between whether or not, or by the addition of one to three amino acids at the N-terminal residue and/or C terminal.

In another embodiment of the present invention, the molecular complex of the invention joining from two to four molecules of the peptide of formula I including modified sequences thereof, as well as the union of said peptides of formula I via a peptide linking or non-peptide.

It is also comprised within the scope of the invention, that referred to the IMMP complex including the Zn ion and use a peptide of formula I, preferably a peptide with theSEQ. ID. No. 1 to SEQ. ID. No. 8, to which the N-terminal and/or C-terminus of the peptide have been blocked.

One application of the analog is to confer stability to the proteolytic degradation.

It is also comprised within the scope of the invention a method for obtaining the complexes of the invention from the peptide of formula I, preferably from the peptide with the SEQ. ID. No. 1 to SEQ. ID. No. 8, since by expressing in biological vectors and adding a divalent ion, such as zinc, the complex of the invention would be integrated.

The physicochemical properties and biological activity of the complex of the present invention are different from that of each components by separate; any non-metallic atom or ion, free, or content in a neutral molecule or in an ionic compound which can transfer a pair of electrons, can act as a donor. The acceptor, ie the component accepting and sharing the pair of electrons is commonly a metal ion. The complex of the present invention may be formed in a range of ratios p/p of zinc: peptide of formula I, from 1 : 0.1 to 1 : 10,000 correspondingly.

The results of nuclear magnetic resonance studies with the IMMP complexes of the invention show that when they interact with the zinc, the carboxylic groups of the glutamic acid (D) changes toward downfield (Δδ + 0.027 ppm), aspartic acid (E) and L terminal carboxylic acid suffer a displacement toward highfield ( Δδ - 0.016 and - 0.051 ppm, respectively). This suggests an interaction of the ion with these groups.

In one of the embodiments of the present invention, includes a method for obtaining the IMMP complex of the invention, characterized in that comprises the steps of:
a) Mixing the peptide of formula I:

   Xn-Asp-Gly-Pro-Lys-Phe-Leu-Xm I

   Where Xn and Xm are any amino acids independently of from another, n has a value of 0 to 4 and m has a value of 0 to 2 , with a buffer solution at pH 7 to 7.5,
b) Adding to the mixture obtained in a) a solution containing a divalent ion selected from the group comprising Zn2+, Cu2+, Mg2+ o or mixtures thereof, and
c) Resting the mixture obtained in b) at 4° C in the dark during enough time until the IMMP complex formation.

In this sense, the IMMP complex of the invention, can be obtained for example taking 1 mmol of peptide of formula I in 1 mL of phosphates buffer (100 mM) at pH of 7.4; subsequently is added drop by drop to this mixture a divalent ion solution, for example for the addition of the Zn divalent ion, a solution of ZnCl2 0.0001 to 1 M, procuring to maintain the pH at 7.2. Once said mixture has been made, it is rested for 14-16 hours at 4° C and in darkness, to subsequently being frozen at -20° C for at least 24 hours, if required, the obtained final mixture is lyophilised.

The present invention is useful in the field of human and veterinary medicine and particularly refers to complexes formed between divalent cations (Zn, Mg, and Cu) and peptides of formula I as novel compounds, the process for obtaining them, their use as medicament, nutraceutical, health promoter, both in humans and animals, as well as their use for manufacturing drugs or nutraceuticals.

The use of the IMMP complex to modify the immune response, reduce inflammation, treat in a preventive, curative or palliative manner an infection, physical or psychological stress, cancer, metastasis, autoimmune disease, fibrosis, thrombocytopenia. The use of the IMMP complex of the invention for producing a therapeutic agent (drug) or nutraceutical for also treating in an animal or human either of the above mentioned conditions, is also comprised.

For purposes of the invention the following terms are used in the context of the invention:
The term "analog peptide" as used herein refers to a peptide that possesses in its sequence at least 50% of the amino acids present in another, in a similar order, it could have the same size, shorter or longer.

The term "nutraceutical" includes substances, chemicals, phytochemicals and biological compounds that can be considered a food or part of a food which can provide medical benefits for the animal and human health. Nutraceutical formulations are comprised within the scope of the invention when in their mixture, one of the active components is any of the metallopeptide molecular complexes of formula I described, either for human or animal use.

The term "secondary immunosuppression", as used herein, refers to the pathological states in which due to some cause such as infection or chemotherapy, among others, the immune response is inefficient.

The term "administration" or any variation thereof (eg. Administrating, administering, etc.), as used herein, refers to any use of an animal to provide the therapeutic or nutraceutical agent and achieve this reaches the site of the organism in which it acts, using the method and/or materials of the invention. Thus, it includes: (i) any way of administration, either general (oral or enteral, parenteral (intravenous, intramuscular, subcutaneous or subdermal), transdermal, inhalation, rectal) or topically (nasal, dermal, etc.), or (ii) indirectly through a biological vector to which it has been transfected with a plasmid having the base sequences encoding the peptide of formula I or some of its variants to produce a recombinant product, as well as any way of inducing the endogenous production of the peptide of formula I.

The term "therapeutically effective amount" as used herein refers to the necessary amount to provoque the desired biological response. A therapeutically effective amount of IMMP for the animal could be between 2 and 2000 ng/kg of body weight, or an equivalent molar amount of the analogues or derivatives, in two or more doses, administered with an interval of 3 to 10 days between one and another, depending on the age, health, or even gender; in the case of animals, it may vary with the specie. Thus, the dose used may be varied according to the species, age, health, or even the gender of the animal.

The pharmaceutical formulations of the invention refer to any chemical and physical form of any of the peptides of formula I described complexed with divalent ions, or to the possible mixtures between each other of said complexes, which contain pharmaceutically acceptable non-active agents and maintain in some ratio or magnitudes the peptide activities of formula I, to which is added a pharmaceutical vehicle, which also confers stability to its storage. Examples of pharmaceutical vfehicles include acidic salts, with an organic or inorganic acid such as acetate, tartrate, trifluoroacetate, lactate, maleate, fumarate, citrate, methane, sulfonate, sulfate, phosphate, nitrate or chloride. To provide the appropriate doses of IMMP of the invention for the desired therapeutic treatment, pharmaceutical or nutraceutical compositions contemplated herein will typically contain between 0.00001 % and 99.999 % w/w of the metallopeptide molecular complex of the invention, including the carrier or diluent.

Generalizing, the term treatment in the context of the invention, refers to the pharmacological handling of a human or animal subject to achieve relief, regression, preventing the progress or course of a health disease or disorder after the subject has developed the disease: thus, it includes: (i) preventing or reducing of an affectation of the conversion index, (ii) reduce the symptoms associated with a biological condition affecting the conversion index (for example, reducing the effects of infection by coccidia or influenza), (iii) preventing, delaying or reducing the presentation of symptoms associated with complications, conditions or diseases associated with the biological condition of interest (eg: preventing, delaying or reducing the presentation of gastrointestinal disorders or diarrhea, coccidiosis or influenza).

The term "co-administration" as used herein, refers to administration of the IMMP along with another agent, for example vaccines, antigens, antibiotics, antivirals, antiparasitics, coccidostatos, etc., which commonly form part of the preventive or curative therapeutic handling scheme on farms, in an equal pharmaceutical or nutraceutical formulation. Therapeutic agents that can be administered with the IMMP include, but are not limited to, anti-inflammatories, antibiotics, antibacterial, agents vs coccidiosis, vitamins, proteins, antibodies, amino acids, lipids, probiotics or prebiotics, etc. Alternatively, the above mentioned agent and the IMMP can be administered concurrently as separate compounds, for example, separated pharmaceutical compositions administered consecutively, simultaneously, or at different times. If the therapeutic agent would include a peptidase or protease enzyme, it should be administered at different times and in different administration sites so that the IMMP and the therapeutic agent cannot have a pharmacological interaction between them and they antagonize each other.

The term "cancer" or any variation thereof (malignant tumor, cancerous tumor) as used herein, refers to a set of diseases characterized by the presence of a tumor or malignant cells accumulation (known as carcinogenic or cancerous), which have generally suffered alterations or transformations in their genetic information, with which they have lost the ability of responding to growth homeostatic controls, proliferation and/or apoptosis, thus, growth and division occur beyond normal limits, invasion of surrounding tissue and sometimes generate distance tumors or metastases, and which threats against life.

The term "immune hypersensitivity" or any variant thereof (for example, hypersensitivity, autoimmunity, allergy), refer to the presence of an immune response against particular molecules, cells and organs, which may occur in any of the four embodiments accepted by the experts in the matter: type I hypersensitivity or anaphylactic, Type II or antibody dependent vs cellular antigens, type III or circulating immune complexes, or type IV or cell dependent or delayed, or a combination thereof.

In this sense, Rager-Zisman et al. (Rager-Zisman, B., 1993) describe the capacity of the THF to increase the immune response against viruses. However, the data shown by Rager-Zisman indicate that higher doses of THF are used than those indicated in the present invention, due to substantial differences in the potency of these compounds.

According to Tables 1, 2, 3, 4, 5 , 6, 8 and 9, our results demonstrate that the IMMP complexes of the present invention are different complexes to those already existing in the prior art, for example the THF native molecule.

It should be understood that the following examples described in the present application are only for illustrative purposes and that several modifications or changes in the context of them may be suggested or performed by persons skilled in the technical field and these will be included within the spirit and purpose of this application.

### Example 1. Zinc effect and other ions, besides the DTPA over the IMMP effects of the invention.

In the literature there is a wide variation in the effective doses reported for LEDGPKFL peptide and since there is the antecendent of that in the thymus the zinc ion is found in abundance, likewise, it has also reported the presence of at least one thymic peptide joining zinc, we decided stimulate THP-1 cells with different concentrations of the peptide, zinc, and peptide + zinc to concentration of 1 micromolar, since the serum concetrations of zinc range between 7.6 to 22 µM. The obtained results are shown in Table 1.

**Table 1. Comparative effect of different LEDGPKFL peptide concentrations, zinc and peptide + zinc (1nM) on the proliferation of THP-1 cells co-stimulated with LPS (0.5 µg/mL).**

| | **Proliferation (% with respect to the control)** | | |
|---|---|---|---|
| **Concentration (M)** | **Peptide LEDGPKFL** | **Zinc (1µM)** | **Peptide LEDGPKFL+Zinc (1µM)** |
| CONTROL | 100 ± 3.4 | 100 ± 3.4 | 100 ± 3.4 |
| -12 | 102 ± 4.3 | 98 ± 4.5 | 108 ± 3.1 |
| -11 | 104 ± 6.9 | 101 ± 3.9 | **140 ± 5.1*^{#}** |
| -10 | 108 ± 8.5 | 100 ± 7.0 | **180 ± 3.9*^{#}** |
| -9 | 110 ± 12.7 | 103 ± 9.9 | **182 ± 4.1*^{#}** |
| -8 | 140 ± 5.4* | 102 ± 12.1 | **178 ± 2.9*^{#}** |
| -7.5 | 166 ± 14.9* | 104 ± 8.8 | 177 ± 8.3* |
| -7 | 170 ± 10.1* | 102 ± 9.7 | 179 ± 7.9* |
| -6 | 173 ± 9.8* | 105 ± 8.8 | 177 ± 10.2* |

| | | | |
|---|---|---|---|
| Each value represents the mean ± SD of three experiments performed by octuplicate. *p>0.01 with respect to the group with LEDGPKFL peptide. ^{#} p>0.01 with respect to the group of the peptide + zinc. Values are expressed as control percent. Effective concentration 50, for the peptide alone, a 10^{-8.1}M , for the peptide for peptide + zinc, 10⁻¹¹ M. | | | |

As shown, the LEDGPKFL peptide stimulated the proliferation of these cells as expected, reaching a plateau in the stimulation from 10-7.5 M (459 ng/mL), zinc ion by itself did not stimulate the proliferation of these cells, while when the cells were incubated in the presence of peptide + zinc (1µM), it was observed that for the concentration of 10-10 M (0.0918 ng/mL) the maximum response was obtained, that is to say, by adding the ion potency was increased almost 500 times.

In order to ascertain if the effect of the addition of the ion was only on the THP-1 cells and also on other cells, human lymphocytes stimulated with PHA were incubated, with different concentrations of the peptide alone, zinc, and peptide + zinc. The results obtained are shown in Table 2.

As can be observed, the zinc ion, by itself did not modify the proliferation of the stimulated cells with PHA, the peptide alone increased the response at higher concentrations of 10-7.5 M, while the same peptide in presence of the zinc ion, was able to do it made from concentrations of 10-10 M, ie, the effect observed in THP -1 cells was repeated.

**Table 2. Comparative effect of different concentrations of LEDGPKFL peptide, zinc and peptide + zinc (10nM) over the proliferation of stimulated human lymphocytes with PHA (1µg/mL).**

| | **Proliferation (% with respect to the control)** | | |
|---|---|---|---|
| **(M) Concentration** | **PHA +Peptide** | **PHA + Zinc (10 nM)** | **PHA + Peptide + Zinc (10 nM)** |
| (PHA) Control | 100 ± 3.1 | 100 ± 3.1 | 100 ± 3.1 |
| -12 | 94 ±3.1 | 96 ± 4.5 | 99 ± 2.2 |
| -11 | 96±7.8 | 99 ± 3.9 | 114 ± 3.6*^{#} |
| -10 | 96±10.4 | 100 ± 3.0 | **159 ± 5.6*** ^{#} |
| -9 | 100±11.9 | 103 ± 3.9 | 154 ± 2.9*^{#} |
| -8 | 107±11.0 | 101 ±4.1 | 156 ± 2.1*^{#} |
| -7.5 | 130±10.6* | 95 ± 3.8 | 158 ± 5.9*^{#} |
| -7 | **154±6.5*** | 99 ± 2.7 | 157 ± 5.6* |
| -6 | 152±7.0* | 96 ± 2.8 | 155 ± 6.1* |

| | | | |
|---|---|---|---|
| Each value represents the mean ± SD of three experiments performed by octuplicate. *p> 0.01 with respect to the control group. ^{#}p >0.01 with respect to the LEDGPKFL peptide group. Values are expressed as control percent. Effective concentration 50, for the peptide alone, 10^{-7.7} M, for the peptide + zinc 10^{-10.8} M. | | | |

To confirm the role of zinc in the immunomodulatory function of the complex of the invention, it was performed tests in THP-1 cells, Jurkat and human lymphocytes, coestimulated the two latter with PHA and adding 5 µM DTPA, a chelator of extracellular zinc. The results obtained are shown in Tables 3, 4 and 5.

**Table 3. Comparative effect of the IMMP and the LEDGPKF8 peptide on the newborn rat thymocyte proliferation coestimulated with A (50 µg/mL).**

| | **Proliferation (% with respect ro the control)** | |
|---|---|---|
| | Con A + Peptide L₁EDPGKFL₈ | With A + IMMP |
| Control (With A) | 100 ± 1.1 | 100 ± 1.1 |
| 10⁻¹² (0.0009 ng/mL) | 104.4 ± 2.5 | **120.3 ± 3.2*^{#}** |
| 10⁻¹¹ (0.009 ng/mL) | 103.7 ± 5.1 | **130.5 ± 1.4*^{#}** |
| 10⁻¹⁰ (0.09 ng/mL) | 104.3 ± 4.8 | **135.6 ± 3.1*^{#}** |
| 10⁻⁹ (0.91 ng/mL) | 110.6 ± 7.9 | **133.3 ± 3.4*^{#}** |
| 10⁻⁸ (9.18 ng/mL) | 117.3 ± 3.2* | **128.3 ± 2.7*^{#}** |
| 10^{-7.5} (9.58 ng/mL) | 119.2 ± 6.1* | 127.4 ± 3.0* |
| 10-⁷ (91.8 ng/mL) | 125.6 ± 3.9* | 125.1 ± 2.6* |
| 10⁻⁶ (918 ng/mL) | 123.1 ± 6.1* | 126.6 ± 3.0 |

| | | |
|---|---|---|
| Each value represents the mean ± SD of three experiments performed by octuplicate. *p> 0.01 with respect to the control group. ^{#}p >0.01 with respect to the LEDGPKFL peptide group. Values are expressed as control percent. Effective concentration 50, for the peptide alone, 10^{-8.6} M, for the peptide + zinc 10^{-12.5} M. | | |

**Table 4. Effect of different concentrations of Zinc over the proliferation of THP-1 cells stimulated with LPS (0.5 µg/mL) and LEDGPKFL peptide (10-10 M) in presence and absence of DTPA.**

| | Proliferation (% with respect to the control) | |
|---|---|---|
| [ZINC] (M) | L₁EDGPKFL₈Peptide **(10⁻¹⁰ M)** | L₁EDGPKFL₈ Péptido **(10⁻¹⁰ M)** + DTPA (5 µM) |
| (LPS) Control | 100 ± 1.6 | 98 ± 5 |
| -12 | 102 ± 5.2 | 101 ± 4.6 |
| -11 | 135 ± 10*^{#} | 104 ± 5.1 |
| -10 | 190 ± 10*^{#} | 104 ± 4.8 |
| -9 | 192 ± 12*^{#} | 105 ± 3.1 |
| -8 | 195 ± 10*^{#} | 103 ± 4.2 |

| | | |
|---|---|---|
| Each value represents the mean ± SD of three experiments performed by octuplicate. *p> 0.01 with respect to the control group. ^{#}p >0.01 with respect to the group with DTPA. Values are expressed as control percent. | | |

As can be shown, leukocytes which received the peptide + zinc increased the phagocytosis up until about four times at 48 hours, but those which besides receiving zinc + peptide also receive DTPA did not increase their phagocytosis.

**Table 5. Effect of different concentrations of Zinc over the proliferation of Jurkat cells stimulated with PHA (1µg/mL) and LEDGPKFL peptide (10-10 M) in presence and absence of DTPA.**

| | Proliferation (% with respect to the control) | |
|---|---|---|
| [ZINC] (M) | L₁EDGPKFL₈ Peptide **(10⁻¹⁰ M)** | L₁EDGPKFL₈ Peptide **(10⁻¹⁰ M)** + DTPA (5 µM) |
| (LPS) Control | 100 ± 1.6 | 98 ± 5 |
| -12 | 102 ± 5.2 | 101 ± 4.6 |
| -11 | 135 ± 10*^{#} | 104 ± 5.1 |
| -10 | 190 ± 10*^{#} | 104 ± 4.8 |
| -9 | 192 ± 12*^{#} | 105 ± 3.1 |
| -8 | 195 ± 10*^{#} | 103 ± 4.2 |

| | | |
|---|---|---|
| Each value represents the mean ± SD of three experiments performed by octuplicate. *p> 0.01 with respect to the control group. ^{#}p >0.01 with respect to the group with DTPA. Values are expressed as control percent. | | |

In the five tests with DTPA (Tables 3, 4, 5, 6 and 7), we observed that the ion presence increases the proliferative response obtained with the peptide alone, but that the chelator of the zinc ion causes the lost of the effect on the cellular proliferation induced by the zinc + peptide.

In order to test a different effect of the proliferation, we decided to investigate the effect of peptide + zinc versus peptide + zinc + DTPA on human leukocytes phagocytosis. The obtained results are observed in Tables 6 and 7.

**Table 6. Effect of different concentrations of Zinc over the proliferation of human lymphocytes stimulated with PHA (1µg/mL) and coestimulated with LEDGPKFL peptide (10-10 M) in presence and absence of DTPA.**

| | Proliferation (% with respect to the control) | |
|---|---|---|
| **[Zinc] (M)** | L₁EDGPKFL₈ Peptide | L₁EDGPKFL₈ Peptide + DTPA |
| (PHA 1 µg/mL) CONTROL | 100 ± 5.1 | 100 ± 4.3 |
| -12 | 98.5 ± 2.2 | 98.5 ± 2.7 |
| -11 | 114.2 ± 3.6*^{#} | 101.4 ± 2.1 |
| -10 | 158.5 ± 5.6*^{#} | 100.0 ± 3.7 |
| -9 | 154.2 ± 2.9*^{#} | 99.2 ± 4.5 |
| -8 | 155.7 ± 2.1*^{#} | 101.4 ± 5.8 |

| | | |
|---|---|---|
| Each value represents the mean ± SD of three experiments performed by octuplicate. *p> 0.01 with respect to the control group. Values are expressed as control percent. ^{#}p >0.01 with respect to the group with DTPA. | | |

**Table 7. Comparative effect of the LEDGPKFL Peptide (10-10 M) + Zinc (10-9 M) on the human leukocytes phagocytosis in presence and absence of DTPA.**

| | | Phagocytosis | |
|---|---|---|---|
| | CONTROL | Peptide + Zinc | Peptide + Zinc + DTPA |
| 24 h | 40±3 | 56±2* | 38±4 |
| 48 h | 43±3 | 185±18* | 44±3 |

| | | | |
|---|---|---|---|
| Each value represents the mean ± SD of four experiments performed by octuplicate. *p> 0.01 with respect to the control group with DTPA. The fluorescence was quantified with a FACSort cytometer of BD, using a logarithmic scale. The data are reported in arbitrary fluorescence intensity units determined by the team. The fluorescence intensity is directly proportional to the amount of materials phagocytosed | | | |

### Example 2. Demonstration of the metallopeptide complex formation of the invention.

To show that the increase in the potency of the peptide of formula I by adding zinc was not an additive effect, but it was the formation of a molecular complex with more power than their predecessors by separated, we proceeded to perform studies of nuclear magnetic resonance.

It was used two Varian Unity Inovacon equipments of frequencies for core of 1 H at 400 MHz and 699.815 MHz at a temperature of 25° C. The spectra were obtained in D2O, and in some cases in mixtures D2O-H2O, using DSS and CDCl3 as external references for 1 H and 13C, respectively.

As shown in Figures 1 to 4, by interacting with the zinc, the carboxylic groups of the glutamic acid (D) change toward downfield (Δδ +0.027 ppm), while that of the aspartic acid (E) and the terminal carboxylic acid of the Leucine (L) at the 8th position of the peptide suffer a displacement toward highfield (Δδ - 0.016 and -0.051 ppm, respectively), which suggests an interaction of the ion with these groups.

### EXAMPLE 3. Study of the effect of other ions on the peptide potency.

We proceeded to perform some related to the proliferative stimulation of the peptide in presence of other divalent cations such as magnesium, copper, and manganese. The obtained results are shown in Table 8.

**Table 8. Effect of the divalent cations Mg++, Cu++, y Mn++ on the THP-1 cell proliferation stimulated with LPS (0.5 µg/mL) and different concentrations of the LEDGPKFL peptide.**

| | Proliferation (% with respect to the control) | | | | |
|---|---|---|---|---|---|
| [LEDGPKFL Peptide] (M) | WITHOUT ION | ZINC | MAGNESIUM | COOPER | MANGANESE |
| CONTROL | 100±3.4 | 100±3.4 | 100±3.4 | 100±3.4 | 100±3.4 |
| -10 | 108±8.5^{#} | **180±3.9*** | 146±8*^{#} | 138±1.1*^{#} | 137±10*^{#} |
| -9 | 110±12.7^{#} | 176±4.1* | **167±10*** | **164±5.1*** | 145±12*^{#} |
| -8 | 140±5.4*^{#} | 178±2.9* | 162±7*^{#} | 167±13* | 150±6*^{#} |
| -7 | **170±10.1*** | 160±7.9* | 158±6* | 160±6* | **162±15*** |

| | | | | | |
|---|---|---|---|---|---|
| It was used a peptide relation: ion of 1: 10. a peptide ratio was used. Each value represents the mean ± SD of three experiments performed by triplicate. *p>0.01 with respect to the to control group. ^{#} p> 0.01 with respect to the group of the peptide + zinc. | | | | | |

As can be observed both magnesium and copper increase the potency of the peptide, but they do it in an magnitude order, that is to say, 10 times less efficiently. Manganese did not modify the potency of the peptide.

We proceeded to investigate if ions modify any other physicochemical parameter of the peptide, such as its polarity, so that we proceeded to submit them to high resolution liquid chromatography of inverse phase, in which the polar molecules firstly elute. The obtained results are shown in Table 9.

As can be observed in that table, the retention time of the peptide alone is only lower when is in the form of molecular complex with a divalent cation, which indicates that these ions decrease the polarity of the peptide, probably it was already observed in the NMR studies and confirming them, by interacting with the carboxylic groups of Glu and Asp. On the other hand, it is observed that the zinc ion neutralizes the peptide in a better way, since the lower retention time is tests.

By the previous results, we conclude that LEDGPKFL peptide and the zinc ion are precursors of a metallopeptide molecular complex with molecular weight of 983.4 Da. The ion interacts with the carboxylic groups of the Glutamic, Aspartic amino acids and the Leucine(8) of the peptide, neutralizing them and modifying somehow the three dimensional structure of the peptide, which favorites its interaction with a receptor, increasing its affinity for it. The zinc ion can be replaced by magnesium and copper ions, but showing a potency at least 10 times lower.

**Table 9. Effect of different ions on the retention time in a column of HPLC of reverse phase.**

| | Control (THF) | Zn | Mg | Mn | Cu |
|---|---|---|---|---|---|
| Time (minutes) | 17.92±0.01 | 17.69±0.06* | 17.88±0.02* | 17.85±0.01* | 17.75±0.12* |

| | | | | | |
|---|---|---|---|---|---|
| Each value represents the mean ± SD of three runs performed by triplicate. * p >0.01 with respect to the control group. | | | | | |

### Example 4. IMMP effect of the invention and of an analogue of the IMMP on newborn rat thymocytes.

Since the reports of that the co-stimulation with Concanavaline A (With-A) and THF induced lymphocyte proliferation of spleen (100 to 300 ng/mL, 109-327 nM) and umbilical cord (300-600 ng/mL, 327 654 nM) and the production of IL-2, we decide to investigate whether the IMMP (LEDGPKFL + zinc) was able to induce the T cells production in the thymus, for which coestimulamostimocitos of newborn rat with 50 µg/mL of With-A and different concentrations of IMMP during 24 hours (see table 10). It was also evaluated the same effect with an IMMP analog, this to determine if the IMMP effects were specific, wherein this analog would modify completely the tertiary structure of the peptide. It was used an octapeptide with similar weight for the elaboration of the complex, to which was just the proline (4) and Glycine (5) original amino acids were interchanged by proline (4) and Glycine (5) resulting as LEDPGKFL. The obtained results are shown in Table 10.

**Table 10. Comparative effect of the IMMP and its LEDPGKFL + Zinc analog on the newborn rat thymocyte proliferation coestimulated with WITH-A (50 1µg/mL).**

| | Proliferation (% with respect to the control) | |
|---|---|---|
| [IMMP] or [Analog] | With A + IMMP | With-A + Analog (L₁EDPGKFL₈) + Zinc |
| Control | 100±2.5 | |
| 10⁻¹² (0.0009ng/mL) | 120.3±±3.2* | 105.3±3.2^{#} |
| 10⁻¹¹ (0.009 ng/mL) | 130.5±1.4* | 108.5±5.4^{#} |
| 10⁻¹⁰ (0.09 ng/mL) | 135.6±3.1* | 110.6±8.1^{#} |
| 10⁻⁹ (0.91 ng/mL) | 123.3±3.4* | 104.3±3.4^{#} |
| 10⁻⁸ (9.18 ng/mL) | 120.3±2.7* | 96.3±2.7^{#} |
| 10⁻⁷ (91.8 ng/mL) | 125.1±2.6* | 98.1±2.6^{#} |
| 10⁻⁶ (918 ng/mL) | 106.6±3.0 | 100.6±3.0 |

| | | |
|---|---|---|
| It was used a relationship analog:Zinc of 1: 10. Values are expressed as control percent. Each value represents the mean ± SD of three experiments performed by triplicate. *p >0.01 with respect to the control group. ^{#}p> 0.01 with respect to the IMMP group. | | |

As can be observed the IMMP (LEDGPKFL + Zinc) increased the thymocytes proliferation stimulated with With-A of newborn rat in an 35% at a concentration of 10-10 M, whereas thymocytes exposed to the LEDPGKFL + zinc analog did not proliferate, which indicated that the proliferation effect is specific for the complex formed by the LEDGPKFL + zinc peptide.

We proceeded to evaluate if the IMMP was able to modify in vitro the newborn rat thymocyte subpopulations. The obtained results are shown in Table 11.

**Table 11. Comparative effect of IMMP and its LEDPGKFL + Zn analog on the expression of the CD4 co-receptor in newborn rat thymocytes co-stimulated With WithA.**

| | **Cells Percent** | | | | | | | |
|---|---|---|---|---|---|---|---|---|
| | With A + IMMP | | | | With A + Analog + Zinc | | | |
| [M] | DN | DP | CD4 | CD8 | DN | DP | CD4 | CD8 |
| Control (With A) | 3.8 ± 1.9 | 81.2 ± 6.2 | 11.2 ± 3.2 | 4.6 ± 0.4 | | | | |
| 10⁻¹² | 6.4 ± 0.4 | **71.8 ± 3.5*** | **1 6.7 ± 0.9*** | 6.0 ± 0.5* | 3.2 ± 0.2^{#} | 82.8 ± 2.9^{#} | 9.7 ± 0.5# | 4.1 ± 0.9^{#} |
| 10⁻¹¹ | 5.1 ± 2.0 | 74.8 ± 5.4 | **1 5.4 ± 0.8*** | 6.0 ± 1.7 | 4.4 ± 1.4 | 77.6 ± 4.0 | 12.3 ± 2.3 | 5.6 ± 1.2 |
| 10⁻¹⁰ | 5.1 ± 1.9 | 73.3 ± 3.0 | **16.7 ± 1.0*** | 4.7 ± 2.2 | 6.2 ± 3.1 | **76.4 ± 5.7** | 12.5 ± 4.5 | 5.1 ± 1.4 |
| 10⁻⁹ | **7.0 ± 1.1*** | **70.5 ± 1.2*** | **17.7 ± 1.9*** | 4.8 ± 1.9 | 6.3 ± 3.4 | **74.7 ± 7.1** | 12.8 ± 5.0 | 5.9 ± 1.5 |
| 10⁻⁸ | 7.1 ± 2.8 | **74.8 ± 0.7*** | **13.9 ± 2.6*** | 6.1± 0.2* | 5.3 ± 2.7 | **77.6 ± 4.5** | 11.3 ± 3.7 | 5.5 ± 0.8 |
| 10-⁷ | 3.7 ± 0.2 | 73.3 ± 2.6 | 14.2 ± 3.7 | **6.0 ± 2.2*** | 5.1 ± 2.6 | **76.3 ± 5.0** | 13.5 ± 3.3 | 5.5 ± 1.2 |
| 10⁻⁶ | 6.6 ± 1.7 | 72.2 ± 1.5 | 15.1 ± 2.5 | 5.3 ± 1.5 | 4.6 ± 3.0 | 77.1 ± 6.0 | 12.8 ± 3.4 | 5.1 ± 1.0 |

| | | | | | | | | |
|---|---|---|---|---|---|---|---|---|
| It was used a relationship analog:Zinc of 1: 10. Values are expressed as control percent. Each value represents the mean ± SD of three experiments performed by triplicate. *p >0.01 with respect to the control group. ^{#}p> 0.01 with respect to the IMMP group. [M] = molar concentration, DN = Double negatives (CD4-CD8-), DP = Double positives (CD4 + CD8 +); CD4 = CD4 + CD8-(single positive) and CD8-CD4 = CD8 + (single positive). | | | | | | | | |

As shown in Table 11, the IMMP modified the percentages of the four thymocytes subpopulations. From the concentration of 10-12 a 10-8 M increased the percentage of CD4 at concentration of 10-7 M increased the percentage of CD8, while the DP cells were reduced to the concentration of 10-12 M and the DN 10-9 M. On the other hand, it was observed that LEDPGKFL analog just reduced the percentage of DP to the concentrations from 10-10 M to 10-8 M. That is to say, it was required 100 times more LEDPGKFL + zinc analog in order that similar modifications were observed. This indicates that the effect is specific for the IMMP complex of the invention.

Since CD4 thymocytes are originated from double positive thymocytes (CD4 + CD8 +) which lose or decrease the expression of the co-receptor CD8, we decided to investigate the effect of different IMMP concentrations and the co-stimulation with With-A (50 ug/ml) on the expression of both CD4 and CD8 co-receptors. The Analysis was performed by flow cytometry, using fluorescence intensity histograms, which allowed us to identify three regions: expression negative, low and high. The obtained results are shown in Table 12. As can be observed the IMMP did not modify the expression of the CD4 co-receptor in newborn rat thymocytes.

**Table 12. IMMP effect on the expression of the CD4 co-receptor in newborn rat thymocytes, coestimulados with With-A.**

| | Expression Intensity of CD4 Fluorescence (mean ± SD) | | |
|---|---|---|---|
| [IMMP] (M) | Negative | Low | High |
| Control (With A) | 5.0 ± 0.0 | 70.7 ± 8.0 | 188.6 ± 6.8 |
| 10⁻¹² (0.0009ng/mL) | 5.0 ± 0.4 | 69.6 ± 7.2 | 194.1 ± 7.3 |
| 10⁻¹¹ (0.009 ng/mL) | 4.5 ± 0.1 | 69.5 ± 7.0 | 196.1 ± 5.4 |
| 10⁻¹⁰ (0.09 ng/mL) | 5.0 ± 0.4 | 69.6 ± 5.2 | 194.1± 10.0 |
| 10⁻⁹ (0.91 ng/mL) | 4.5 ± 0.1 | 69.5 ± 5.0 | 196.1 ± 5.4 |
| 10⁻⁸ (9.18 ng/mL) | 4.7 ± 0.3 | 71.0 ± 6.4 | 181.1 ± 4.7 |
| 10⁻⁷ (91.8 ng/mL) | 4.5 ± 0.9 | 68.6 ± 8.0 | 188.4 ± 4.9 |
| 10⁻⁶ (918 ng/mL) | 5.0 ± 0.9 | 72.9 ± 7.4 | 193.0 ± 3.3 |

| | | | |
|---|---|---|---|
| Each value represents the mean ± SD of three experiments performed by triplicate. *p>0.01 with respect to the control group. The fluorescence was quantified with a FACSort of BD cytometer, using a logarithmic scale. The data are reported in arbitrary fluorescence intensity units determined by the equipment. | | | |

The IMMP, at low concentrations (10-12 M and 10-11 M) increased the expression of the CD8 co-receptor, but at higher concentrations (10-10 M to 10-6) M decreased its expression (see Table 13). The previous indicates that at low concentrations it favors the differentiation towards subpopulation of CD8+ thymocytes and at high concentrations favors the differentiation towards subpopulation of CD8 + thymocyte.

**Table 13. Effect of different IMMP concentrations on the expression of the CD8 co-receptor in newborn rat thymocytes co-stimulated with With-A.**

| | Expression Intensity of CD8 Fluorescence (mean ± SD) | | |
|---|---|---|---|
| | Negative | Low | High |
| Control (With A) | 2.3 ± 0.0 | 64.7 ± 4.2 | 156.6 ± 0.0 |
| 10⁻¹² (0.0009ng/mL) | 2.0 ± 0.4 | 58.1 ± 3.2 | **194.1 ± 7.3*** |
| 10⁻¹¹ (0.009 ng/mL) | 3.5 ± 0.1 | 56.5 ± 4.0 | **196.1 ± 5.4*** |
| 10⁻¹⁰ (0.09 ng/mL) | 4.1 ± 0.4 | **45.8 ± 3.2*** | **126.5± 7.3*** |
| 10⁻⁹ (0.91 ng/mL) | 3.2 ± 0.1 | **32.7 ± 5.0*** | **139.8 ± 5.4*** |
| 10⁻⁸ (9.18 ng/mL) | 3.2 ± 0.3 | **39.2 ± 5.4*** | **126.1 ± 4.7*** |
| 10⁻⁷ (91.8 ng/mL) | 2.4 ± 0.9 | 55.1 ± 4.9 | **134.5 ± 4.9*** |
| 10⁻⁶ (918 ng/mL) | 3.1 ± 0.5 | 49.9 ±37.4 | **135.9 ± 3.3*** |

| | | | |
|---|---|---|---|
| Each value represents the mean ± SD of three experiments performed by triplicate. *p>0.01 with respect to the control group. The fluorescence was quantified with a FACSort of BD cytometer, using a logarithmic scale. The data are reported in arbitrary fluorescence intensity units determined by the equipment | | | |

Example 5. IMMP effect of the invention on the spontaneous apoptosis of thymocytes, survival of thymocytes and lymphocytes to the treatment with cortisone.

According to the previous results, and considering that the IMMP should have a physiological role in the thymus, and that in the thymus mainly two processes are carried out, positive selection and negative selection; the previous results showed us that the IMMP can participate in positive selection (increase of the thymocytes proliferation and modification of the expression of the CD8 co-receptor), thus, we decided to investigate whether the IMMP was also able to modify the spontaneous apoptosis in this organ (negative selection). For that, we made organ culture in agarose gels and a filter with pores of 5 micron to prevent migration of the thymocytes. The Apoptosis was quantified with 7AAD and it was evaluated by flow cytometry. The obtained results are shown in Table 14.

**Table 14. IMMP effect on spontaneous apoptosis in the thymus.**

| [IMMP] | Thymocytes in apoptosis (%) |
|---|---|
| **CONTROL** | 3.6 ± 0.3 |
| **10⁻¹¹** | 2.5 ± 0.91 |
| **10⁻¹⁰** | 1.5 ± 0.0 9* |
| **10⁻⁹** | 1.7 ± 0.7* |
| **10⁻⁸** | 1.6 ± 0.8* |

| | |
|---|---|
| Each value represents the mean ± SD of three experiments performed by triplicate. *p>0.01 with respect to the control group. | |

Since we had already observed that the IMMP favored the lymphocytes proliferation stimulated with PHA and THP-1 cells, and as it is known cortisone destroys these ones, we decided to investigate if the IMMP protects THP-1 cells from the toxic action of the corticoid, for this we incubated these cells with different concentrations of cortisone and IMMP (10-10 M). The obtained results are shown in Table 16.

**Table 16. IMMP effect on the viability of THP-1 cells incubated with cortisone.**

| | Cell Viability (% with respect to the control) | |
|---|---|---|
| CONTROL | 100 ± 6 | |
| IMMP (10⁻¹⁰ M) | 180 ± 5 | |
| | Cortisone | Cortisone± IMMP (10⁻¹⁰ M) |
| 10⁻⁸ | 80 ± 6* | **180 + 5*^{#}** |
| 10⁻⁷ | 70 ± 7* | **155 + 8*^{#}** |
| 10⁻⁶ | 65 ± 5* | **145 + 6*^{#}** |

| | | |
|---|---|---|
| Each value represents the mean ± SD of three experiments performed by triplicate. *p>0.01 with respect to the control group # p>0.001 with respect to the cortisone group without IMMP. Values are expressed as control percent | | |

As can be observed, the effect of the IMMP, not only protects from the deth induced by the cortisone, but it induced cell proliferation, observing higher values than that observed when cells are exposed only to the IMMP.

### Example 6. IMMP effect of the invention on the production of antibodies against vaccines.

In a broiler farm two groups were created within the same booth, a control group constituted by 43,100 chickens (21,600 females and 21,500 males) and a group treaedy with IMMP ( 200ng/Kg ) constituted by 25,800 animals (11,500 females and 14,300 males). Animals received two doses of IMMP (200ng/Kg) to the day 13 after birth, together with Newcastle vaccine (attenuated virus) and ten days later, along with a bacterin. It was taken 20 chickens of each group (10 females and 10 males) every week and it was monitored the presence of antibodies against Newcastle weekly. The obtained results are shown in Table 17.

**Table 17. IMMP effect on antibody production against Newcastle in chickens.**

| **GROUP** | **Day 13** | **Day 23** | **Day 30** | **Day 37** |
|---|---|---|---|---|
| **CONTROL** | 0 | 22.8 | 38.0 | 59.6 |
| **IMMP (10 ng/dose)** | 0 | 18.0 | **64.5** | **60.2** |

| | | | | |
|---|---|---|---|---|
| The results are expressed as the number of times that it was possible the serum dilution and observe the 100% of the response. | | | | |

As can be observed, the joint administration of the vaccine vs. Newcastle and IMMP produced an acceleration in the total antibodies production vs. Newcastle, so that by the day 37 of the first joint administration, the group that received the treatment has almost the double of antibodies than the group only received the vaccine, said value was reached by this group almost a week later. Although the final levels of antibody did not significantly differ from control.

### Example 7. Effect of the pretreatment with IMMP on inflammation (acute hepatitis).

With the objective of determining whether pretreatment with IMMP modifies acute toxic hepatitis caused by the administrating of CCl4, to 24, 48 and 72 hours, 72 male Wistar rats were divided into 3 groups: each for the mentioned time period, likewise each group was subdivided into four subgroups: control, IMMP, CCl4, CCl4 + IMMP. It was administered three doses of IMMP of the invention (100 ng/kg) to the animals via intraperitoneally, on days 1, 3 and 5 of the test. The Day 8 it was orally administered CCl4 (4g/kg).

The animals were sacrificed at 24, 48 and 72 hours, depending the group to which they belonged. The animal and the liver was weighed, and samples of liver tissue and blood for histological and biochemical determinations were taken. The obtained results are shown in Table 18.

**Table 18. Effect of the pretreatment with IMMP on the relative weight of the liver of rats with acute hepatitis induced with CCl4.**

| **Time (hours)** | **Control** | **IMMP** | **CCl₄ 4** | **CCl₄ + IMMP 4** |
|---|---|---|---|---|
| 24 | 5.2 ± 0.6 | 5.4 ± 0.5 | 8.6 ± 0.7 | 8.0 ± 0.9 |
| 48 | 4.8 ± 0.6 | 5.0 ± 0.4 | 8.4 ± 0.6 | **6.8 ± 0.7*^{#}** |
| 72 | 5.0 ± 0.3 | 4.9 ± 0.2 | 7.7 ± 1.0 | **5.7 ± 0.5*^{#}** |

| | | | | |
|---|---|---|---|---|
| Values represent the mean + SD of two experiments performed by quadruplicate. The values are expressed in relative units of dividing the organ weight by the weight of the animal. | | | | |

As can be observed, pretreatment with IMMP produced a reduction in the relative weight of the liver at 48 and 72 hours after exposure to CCl4, which suggests that the IMMP produced a liver protection against damage induced by CCl4.

Glutathione is a protective molecule of the hepatocyte, its function is to capture free radicals and prevent lipid peroxidation. Since this molecule is one of the mechanisms of protection against CCl4, we investigated the IMMP effect on their hepatic levels. The obtained results are shown in Table 19.

**Table 19. Effect of the pretreatment with IMMP on the hepatic glutathione of rats with acute hepatitis induced by CCl4.**

| **Time (hours) Control** | **(ng/mg of tissue) IMMP** | **(ng/mg of tissue) CCl₄** | **(ng/mg of tissue) CCl₄ + IMMP** |
|---|---|---|---|
| 24 | 3.1 ± 1.0 | 3.7 ± 0.5 | 13.3 ± 3.3*^{#} |
| 48 | 3.5 ± 0.3 | 3.1 ± 0.8 | 14.2 ± 2.8*^{#} |
| 72 | 5.1 ± 0.6 | 9.0 ± 1.5 | 3.7 ± 1.6^{#} |

| | | | |
|---|---|---|---|
| Values represent the mean + SD of two experiments performed by quadruplicate. *p>0.01 with respect to the control group, # p>0.01 with respect to the CCl₄ group. | | | |

As can be observed, the pretreatment of animals with IMMP increased the glutathione production from 24 hours after the exposure to CCl4, which indicates that indeed the IMMP induces liver protection against the induced liper-oxidation by the toxicant agent and its metabolites through inducing antioxidant defense system, preventing membrane lipid peroxidation.

On the other hand, we observed the effect of pretreatment with IMMP on blood levels of liver enzymes. The obtained results are shown in Table 20.

**Table 20. Effect of pretreatment with IMMP on levels of serum ALT, AST and PA in rats with acute hepatitis induced with CCl4.**

| Time of CCl₄ (Days) | Enzyme | Control | IMMP | Acute Hepatitis | Acute Hepatitis + IMMP |
|---|---|---|---|---|---|
| 24 | ALT (UI/L) | 84.7 ± 22.9 | 64.0 ± 22.4 | **320.8 ± 4.6*** | **351.0 ± 10.4*^{#}** |
| 48 | | 68.2 ± 7.7 | 73.1 ± 6.1 | **292.4 ± 16.9*** | **275.54 ± 22.3*** |
| 72 | | 77.4 ± 10.0 | 71.4 ± 8.4 | **269.5 ± 23.2*** | **233.0 ± 20.8*** |
| 24 | AST (Ul/L) | 69.5 ± 8.5 | 72.4 ± 4.1 | **446.3 ± 50.4*** | **493.6 ± 30.3*** |
| 48 | | 67.3 ± 6.9 | 72.5 ± 3.9 | **379.2 ± 8.5*** | **377.0 ± 17.7*** |
| 72 | | 69.9 ± 5.5 | 71.6 ± 6.9 | **276.4 ± 34.8*** | **230.2 ± 15.4*** |
| 24 | PA (UI/L) | 698.5 ± 47.9 | 525.0 ± 160 | **2035 ± 118.9*** | **1139.0 ± 86**.**8***^{#} |
| 48 | | 632.4 ± 49.1 | 804.5 ± 39.5 | **2335 ± 196.6*** | **1287.5 ± 83.6*^{#}** |
| 72 | | 610.4±34.7 | 604.0 ± 26.9 | **2938 ± 83.1*** | **1306.2 ± 90.7*^{#}** |

| | | | | | |
|---|---|---|---|---|---|
| Values are expressed as mean ± SD of 2 experiments performed by quadruplicate. * p >0.01 with respect to the control group, # p>0.01 with respect to the hepatitis group.. ALT = alanine amino transferase, AST = aspartate aminotransferase, PA = alkaline phosphatase. | | | | | |

As can be observed, although the animals pretreatment with the IMMP in rats with acute hepatitis induced with CCl4 produced an increase in liver enzymes ALT and AST to 24 after injury, the activity of these enzymes was reduced after this time. However, animals pretreatment with the IMMP in rats with acute hepatitis reduced the PA activity since said 24 hours. This suggests that the IMMP protected animals from the damage induced by CCl4, reducing inflammation and producing a lower obstruction of the bile canaliculi.

### Example 8. IMMP effect of the invention on stress.

It was administered dead bacteria intraperitoneally at different groups of rats, to simulate a septic shock and physical stress. Later, a group of them was administered with 10 ng/dose of IMMP intraperitoneally. The obtained results are shown in Table 21.

**Table 21. Effect of IMMP administering on body weight, serum levels of IL-1 and corticosterone in a stress model induced in mice.**

| | **CONTROL** | **STRESS** | **STRESS** + **IMMP** |
|---|---|---|---|
| WEIGHT (g) | 14.0 ± 1.1 | 9.3 ± 1.1* | 13.8 ± 1.2^{#} |
| IL-1 (ng/dL) | 7.2 ± 1.0 | 24.6 ± 3.8* | 6.9 ± 0.8^{#} |
| corticosterone (ng/dL) | 32,3 ± 2.7 | 55.4 ± 4.6* | 33.5 ± 2.1^{#} |

| | | | |
|---|---|---|---|
| Values represent the mean + SD of two experiments performed by quadruplicate. * p>0.01 with respect to the control group, # p >0.01 with respect to the stress group. | | | |

As can be observed, the administration of the IMMP reduced the body weight loss of 36% to 11%. It is known that during stress the production of IL-1 is increased, responsible among other things of the fever and malaise in general. We measured plasma concentration of this cytokine and we observed that as in the group with stress the control value was increased three times, while in the group of animals that received the IMMP, this cytokine was increased only once. On the other hand, the IMMP also reduced the increase in corticosteroid production induced by stress, from 70% to 38% (almost 50%). This joint action of reducing plasmatic levels of pro-inflammatory cytokines and corticoids, causes that systemic inflammation is lower, and prevents immunosuppression.

### Example 9. IMMP effect of the invention on cancer.

In order to determine whether the IMMP can protect immunocompetent mice against the tumors development, it is common the use of immunodeficient mice for this purpose, but by contrary we decided using animals with the full immune system. Lymphoma LR.4 cells were inoculated intraperitoneally to mice of the C57BL/6J strain. The LR.4 cells produce a liquid tumor that cannot be measured; however, there is a weight increase in mice as the tumor grows; further, they trigger an immune response against the tumor.

Two weeks after the implantation of tumor cells in groups of mice, in which an weight increase was already observed in the animals with tumor, it was started the administering of IMMP (200 ng/Kg), intraperitoneally. Ten doses were administered, five per week, in a total of two weeks. The weight of the animals was monitored. The obtained results are shown in Table 22.

**Table 22. Effect of IMMP administering on body weight of C57BL/6J mice with lymphoma LR.4.**

| | CONTROL | IMMP | STRESS | STRESS + IMMP |
|---|---|---|---|---|
| WEIGHT (g) | 14.0 ± 1.1 | 13.8 ± 1.2 | 9.3 ± 1.1* | **13.1 ± 0.9*^{#}** |
| IL-1 (ng/dL) | 7.2 ± 1.0 | 6.9 ± 0.8 | 24.6 ± 3.8* | **18.1 ± 2.0*^{#}** |
| Corticosterone (ng/dL) | 32.3 ± 2.7 | 33.5 ± 2.1 | 55.4 ± 4.6* | **43.1 ± 3.8*^{#}** |

| | | | | |
|---|---|---|---|---|
| Values are expressed as mean ± SD of 1 experiment performed by sixfold. Values are expressed as control percent. * p >0.01 with respect to the control group, # P> 0.01 with respect to the tumor group. | | | | |

As can be observed, the IMMP by itself does not modify the body weight of the animals, whereas mice to which that the tumor was inoculated showed a weight increase becoming greater than 25 % to the control. While the group to which the tumor was inoculated and received treatment with IMMP, showed an increase of initial weight, which indicates that the tumor was developing and from day 20, five days after the initiation of the treatment, it was noted a decrease in weight relative to the previous measurement, and with the untreated group. Ten days later, to the thirty day, the weight of animals with tumor was 25% higher than that of the control, whereas that which in fact received the treatment was almost equal to that of the control.

We decided to evaluate the IMMPeffect on LR.4 lymphoma growth at long term, without modifying the treatment scheme to know if the IMMP effect persisted. The obtained results are shown in Table 23.

**Table 23. Effect of the IMMP administering at long term on the body weight of C57BL/6J mice with lymphoma LR.4.**

| | **Time (Weeks)** | | | | | | | | |
|---|---|---|---|---|---|---|---|---|---|
| | 0 | ±4 | 8 | 12 | 16 | 20 | 24 | 28 | 32 |
| CONTROL | 100 ±5 | 100±3 | 100 ± 6 | 100 ± 5 | 100 ± 4 | 100 ± 5 | 100 ± 4 | 100 ± 6 | 100±4 |
| IMMP | 100 ±3 | 102±4 | 101±3 | 99 ± 4 | 101 ± 4 | 102 ± 3 | 100 ± 3 | 100 ± 2 | 100±5 |
| LR.4 | 100 ±3 | 124±5* | 115±4* | 136±6* | 141 ± 8* | 146±11* | 155±9* | 146±10* | 143±15* |
| LR.4 + IMMP | 100 ±2 | 115±5*^{#} | 106±3^{#} | 118±7*^{#} | 120±9*^{#} | 119±9*^{#} | 120±8*^{#} | 119±11*^{#} | 120±13*^{#} |

| | | | | | | | | | |
|---|---|---|---|---|---|---|---|---|---|
| Values are expressed as mean ± SD of 1 experiment performed by sixfold. Values are expressed as control percent. * p >0.01 with respect to the control group, # p> 0.01 with respect to the tumor group. | | | | | | | | | |

As can be observed, in the group with tumor of LR.4, it was observed a tumor decrease between eight and ten weeks, but latter it was increased up until being of about 40% of the body weight. At week 32, mice with tumor had serious physical impairment, so it was decided to terminate the study. On the other hand, it was observed that the administering of IMMP in 10 doses, for two weeks, reduced tumor growth at long by about 50%, from the fourth week of treatment. It was also observed that the IMMP improved living conditions of animals, since the fact of reducing the body weight allowed having a greater mobility, and increased access to water and food.

We performed a similar study to the first, but this time we used mice of the BALB/c strain to which we know that tumor by LR.4 cells kills in a short time period since its immune response against the tumor is not good. The obtained results are shown in Table 24.

**Table 24. Effect of the IMMP administering on body weight of BALB/c mice with LR.4lymphoma.**

| | Time (Weeks) | | | | | | | |
|---|---|---|---|---|---|---|---|---|
| | 0 | 1 | 2 | 3 | 4 | 5 | 6 | 7 |
| CONTROL | 100 ± 3 | 100 ± 2 | 100 ± 4 | 100 ± 3 | 100 ± 2 | 100 ± 5 | 100 ± 4 | 100 ± 5 |
| IMMP | 100 ± 2 | 100 ± 4 | 100 ± 5 | 100 ± 3 | 100 ± 3 | 100 ± 4 | 100 ± 3 | 100 ± 5 |
| LR.4 | 100 ± 4 | 104 ± 3 | 120 ± 6* | 124 ± 7* | 132 ± 7* | 160 ± 10* | 170 ± 10* | 175 ± 13* |
| LR.4 + IMMP | 100 ± 2 | 101 ± 4 | 116 ± 6* | 125 ± 8* | 126 ± 8* | 154 ± 11* | 152 ± 12* | 150 ± 11*^{#} |

| | | | | | | | | |
|---|---|---|---|---|---|---|---|---|
| Values are expressed as mean ± SD of 1 experiment performed by sixfold. Values are expressed as control percent. * p >0.01 with respect to the control group, # p> 0.01 with respect to the tumor group. | | | | | | | | |

As can be seen, treatment with IMMP decreased the tumor growth in the last two weeks, up to about 33%. The test was suspended because the group with tumor had a higher mortality at 50%.

For the above described test results, we studied the IMMP effect on the survival of BALB/c mice with LR.4 lymphoma. The obtained results are shown in Table 25.

**Table 25. Effect of the IMMP administering on the survival of BALB/c mice with LR.4 lymphoma.**

| | Time (Weeks) | | | | | | | | | | | | | | |
|---|---|---|---|---|---|---|---|---|---|---|---|---|---|---|---|
| | 0 | 1 | 2 | 3 | 4 | 5 | 6 | 7 | 8 | 9 | 10 | 11 | 12 | 13 | 14 |
| CONTROL | 100 | 100 | 100 | 100 | 100 | 100 | 100 | 100 | 100 | 100 | 100 | 100 | 100 | 100 | 100 |
| IMMP | 100 | 100 | 100 | 100 | 100 | 100 | 100 | 100 | 100 | 100 | 100 | 100 | 100 | 100 | 100 |
| LR.4 | 100 | 100 | 100 | 100 | 84 | **67** | **50** | **50** | **50** | **34** | **34** | **34** | **16** | 0 | 0 |
| LR.4 + IMMP | 100 | 100 | 100 | 100 | 100 | 100 | 100 | 100 | 100 | **67** | **50** | **34** | **16** | **16** | 0 |

| | | | | | | | | | | | | | | | |
|---|---|---|---|---|---|---|---|---|---|---|---|---|---|---|---|
| Values are expressed as mean ± SD of 1 experiment performed by sixfold. Values are expressed as percent of live animals at the time of counting. | | | | | | | | | | | | | | | |

As can be seen, IMMP administration to animals with tumor kept alive all the animals with LR.4 cells for 5 weeks more than the untreated group, and the survival at 50%, was greater than 4 weeks. Besides that the general conditions of the animals were much better.

As we knew that LR.4 cells have a tumor antigen on its surface and that it is possible to find antibodies in mouse plasma, we performed a titration assay with the serum of the animals, in order to know which group had more quantity of antibodies against tumor. This was performed by flow cytometry. LR.4 cells were washed and incubated in serum dilutions obtained from mice, using as second antibody an antibody vs. mouse conjugated to fluoresce. The obtained results are shown in Table 26.

**Table 26. Effect of the IMMP administering on the antibody levels vs. LR.4 cells in C57BL/6J mice with in LR.4 lymphoma.**

| **DILUTION** | 0 | 1:2 | 1:4 | 1:8 | 1:16 | 1:32 | 1:64 |
|---|---|---|---|---|---|---|---|
| **CONTROL** | 0 | 0 | 0 | 0 | 0 | 0 | 0 |
| **IMMP** | 0 | 0 | 0 | 0 | 0 | 0 | 0 |
| **LR.4** | 100 | **100** | 82 | 50 | 25 | 0 | 0 |
| **LR.4 + IMMP** | 100 | 100 | 100 | **100** | 83 | 40 | 15 |

| | | | | | | | |
|---|---|---|---|---|---|---|---|
| Values are expressed as the number of dilutions performed and the% of cells stained with the antibody. | | | | | | | |

As can be observed, mice with tumor and treated with IMMP showed four times more antibodies than those of the untreated group.

### Example 10. IMMP effect of the invention on metastasis.

We investigated whether treatment with IMMP, modified the metastasis presence of LR.4 cell to different organs. Thymus and spleen were studied since they correspond to lymphatic organs, liver which corresponds to the main organ to which the metastases of abdominal tumors and kidney are directed are directed by their high blood irrigation. The obtained results are shown in Table 27.

**Table 27. Effect of the IMMP administering on the presence of metastasis in C57BL/6J mice with LR.4 lymphoma.**

| Metastases | | | | |
|---|---|---|---|---|
| | Spleen | Thymus | Kidney | Liver |
| **LR.4** | 0/5 | 0/5 | 0/5 | 3/5 |
| **LR.4 + IMMP** | 0/5 | 0/5 | 0/5 | 0/5 |

| | | | | |
|---|---|---|---|---|
| Values are expressed as the number of animals with metastases detected/total number of animals. | | | | |

As shown, 60% of animals with LR4 lymphoma showed metastasis in liver. However, none of the animals with LR4 lymphoma treated with IMMP showed metastases in that organ. That is to say, the IMMP treatment prevented the metastases development.

We evaluated whether the IMMP modifies the growth of a solid tumor, as well as the metastasis generation. We used BMK-16myc cells which are baby mouse kidney cells immortalized with genes of 16 protein of the human papilloma virus (HPV) and the myc gene, which grow in mouse, are highly invasive and produce metastases rapidly. One million cells were implanted subcutaneously in the back of each mouse. The animals were divided into two groups, the first one which had the tumor and did not receive any treatment, and the second to which the tumor was implanted and one week later it was administered 10 doses intraperitoneally of IMMP (200 ng Kg) with the same treatment scheme previously described. Subsequently, the animals were sacrificed and the tumors under the back skin as well as thymus, spleen, liver and kidney were collected for histopathological examination. Tumors Measuring between both groups showed a tendency to that tumors of the treated group with IMMP were smaller; however the difference was not statistically significant. The obtained results are shown in Table 28.

**Table 28. Effect of the IMMP administering of on the metastasis presence in mice with tumors induced by the inoculating of BMK-16myc cells.**

| **Group** | **Spleen** | **Thymus** | **Kidney** | **Liver** |
|---|---|---|---|---|
| **Tumor** | 0/5 | 0/5 | 0/5 | 5/5 |
| **Tumor + IMMP** | 0/5 | 0/5 | 0/5 | 3/5 |

| | | | | |
|---|---|---|---|---|
| Values are expressed as the number of animals with detected metastases/total number of animals. | | | | |

As can be observed, the animals with tumor induced by the inoculation of BMK/16myc cells showed metastases in the liver (100%), however, animals with tumor induced by the inoculation of BMK/16myc cells treated with IMMP showed only metastases in liver at 60%. That is to say, the treatment reduced the development of metastasis in 40% of animals. On the other hand, studying spleen cuts, it was observed, in animals treated with IMMP, the presence of large germinal cores, without evidence of metastasis, which suggests a greater immune response.

To investigate if the IMMP had any direct cytotoxic effect on tumor cells used in the previous examples, and it was for that reason that the described effects were observed, it was cultured the LR.4 and BMK-16myc cells in the presence and absence of IMMP at different concentrations. The cellular viability that was quantified by the MTT method, indicates the mitochondrial activity. The obtained results are shown in Table 29.

As can be observed, the IMMP increased in 20% the LR.4 cells, that is to say, it stimulated its proliferation. In the case of BMK-16myc cells it was not observed any difference with respect to control. This experiment indicated us that the IMMP has no toxic activity for any of the two types of studied cells, which suggests that in vivo there is an indirect mechanism through which the IMMP inhibits cell proliferation, as well as their migration or implantation to generate metastases.

**Table 29. Effect of the IMMP administering on the LR.4 and BMK-16myc cells proliferation.**

| **IMMP (M)** | **LR.4** | **BMK-16myc.** |
|---|---|---|
| **CONTROL** | 100 ± 3.4 | 100 ± 2.4 |
| -12 | 104 ± 4.3 | 102 ± 3.1 |
| -11 | 110 ± 10.9* | 103 ± 2.7 |
| -10 | 120 ± 8.5* | 102 ± 3.5 |
| -9 | 118 ± 6.7* | 104 ± 3.1 |
| -8 | 115 ± 5.4* | 102 ± 2.9 |

| | | |
|---|---|---|
| Values are expressed as mean ± SD of 3 experiments performed by sixfold. Values are expressed as control percent. * p >0.01 with respect to the control group. | | |

### Example 11: IMMP Effect on rheumatoid arthritis in rats.

As we noted that the IMMP has a potential therapeutic use against autoimmune hepatitis, we wanted to know whether the observed effect was exclusive in that pathology or it was possible to observe it in another autoimmune disease. We performed a test of the IMMP effect on rheumatoid arthritis in rats, using a model described in the literature, which consists in administering, two times type I collagen of bovine origin at the base of the rat tail. This produces that in the third week appears edema of the cephalic and caudal extremities, which can be measured with a vernier. Inflammation is due to the presence of the own autoimmune response against type I collagen of the rat. The obtained results are shown in Table 30.

**Table 30: Effect of the IMMP administering on inflammation of the palmar and plantar pad of rats with rheumatoid arthritis.**

| | Inflammation 100 % | Inflammation 50 % | Inflammation 0 % |
|---|---|---|---|
| Control | 0 | 0 | 0 |
| IMMP | 0 | 0 | 0 |
| AR | 12 | 0 | 0 |
| AR + IMMC | 6 | 3 | 3 |

| | | | |
|---|---|---|---|
| The values indicate the number of rats showing the referred inflammation degree in the column. It was considered the 100% the mean of the difference between the diameter of the extremity of animals with AR and the control ones. | | | |

As can be seen, treatment with the IMMP reduced inflammation at 100% in 25% of animals, and at 50% in another 25%, while it did not modify inflammation of the half of the animals. That is to say, the IMMP reduces inflammation in rheumatoid arthritis in rats.

### Example 12. IMMP effect of the invention on platelet levels.

Performing In vivo studies in rats, we observed that the IMMP administering in normal animals produces an increase in blood platelets, of almost 100% (Table 31).

**Table 31: Effect of the IMMP administering on platelet levels in normal rats.**

| Group | Platelets 10³/µL |
|---|---|
| Control | 757 ± 197 |
| Vehicle | 767 ± 307 |
| IMMP | **1560 ± 248*** |

| | |
|---|---|
| Values are expressed as mean ± SD of two experiments performed in sixfold. * p >0.001 with respect to the control group. | |

The platelets increase may be beneficial for an individual or otherwise, representing a risk of thrombosis. We decided to evaluate the treatment effect with IMMP on a process that induces thrombocytopenia, and that in the hepatic diseases the level of platelets is affected, we pretreated animals with IMMP, to the dose of 50 ng/kg, each third day, three dosages, and subsequently we induced acute toxic hepatitis administering CCl4. The obtained results are shown in Table 32.

**Table 32: Effect of the IMMP administering on secondary thrombocytopenia to acute toxic hepatitis.**

| GROUP | Platelets 10³/uL |
|---|---|
| CONTROL | 757 + 197 |
| IMMP | 1560 + 248* |
| CCl₄ | 413 + 92* |
| CCl₄ + IMMP | **886 + 110^{#}** |

| | |
|---|---|
| Values are expressed as mean ± SD of two experiments performed by sixfold. * p >0.001 with respect to control group, # p >0.001 with respect to hepatitis group. | |

As can be observed the pretreatment with IMMP prevented the decrease in platelet blood levels, presented in animals with toxic damage of the liver.

### References.

1. Afdhal N, McHutchison J, Brown R, Jacobson I, Manns M, Poordad F, Weksler B, Esteban R. (2008). "Thrombocytopenia associated with chronic liver disease." J Hepatol. 48(6): 1000-7.
2. Ahmadzadeh M, Rosenberg S. (2005). "TGF-beta 1 attenuates the acquisition and expression of effector function by tumor antigen-specific human memory CD8 T cells." J. Immunol. 174: 5215-5223
3. Bach JF, Dardenne, M. & Pleau, J. M. Biochemical characterisation of a serum thymic factor Nature 266, 55-57 (1977).
4. Bach JF, Hamburger J. (1979) "Polypeptide possessing thymic activity" US4133804(A)
5. Barak Y, H. T., Pecht M, Karov Y, Berrebi A, Zaizov R, Stark B, Buchner V, Burstein Y, Trainin N. (1992). "Thymic humoral factor-gamma 2, an immunoregulatory peptide, enhances human hematopoietic progenitor cell growth." Exp Hematol. 20(2): 173-7.
6. Barbour, et. al. (1998) Humoral and cell-mediated immunopotentiation in vaccinated chicken layers by thymic hormones and zinc. Vaccine. 16(17):1650-1655.
7. Bataller R, Schwabe RF, Choi YH, Yang L, Paik YH, Lindquist J, et al. (2003). "NADPH oxidase signal transduces angiotensin II in hepatic stellate cells and is critical in hepatic fibrosis." J Clin Invest 112: 1383-94.
8. Batts KP, Ludwig J. (1995). "Chronic hepatitis. An update on terminology and reporting." Am J Surg Pathol 19: 1409-17.
9. Be'nichou C. (1990). "Criteria of drug-induced liver disorders. Report of an international consensus meeting." J Hepatol 11: 272-6.
10. Benjamini E, Sunshine G, Leskowitz S. (1996). Immunology. A short course. USA, Wiley-Liss Publishing.
11. Berinstein NL (2009). "Strategies to enhance the therapeutic activity of cancer vaccines: using melanoma as a model." Ann N Y Acad Sci 1174: 107-17.
12. Bhattacharya SK, Brown MF, Dorf PH, La GrecaSD, Maguire RJ. (2009) Cycloalkylamino acid derivatives and pharmaceutical compositions thereof. RSP20080525(A)
13. Borysiewicz LK, Fiander A, Nimako M, Man S, Wilkinson GW, Westmoreland D, Evans AS, Adams M, Stacey SN, Boursnell ME, Rutherford E, Hickling JK, Inglis SC. (1996). "A recombinant vaccinia virus encoding human papillomavirus types 16 and 18, E6 and E7 proteins as immunotherapy for cervical cancer." Lancet 347(9014): 1523-7.
14. Burstein Y, Trainin N. et. al. (1999). "THF-gamma2 analogs and phamaceutical compositions comprising them". US5968898
15. Burstein Y, B. V., Pecht M, Trainin N. (1988). "Thymic humoral factor gamma 2: purification and amino acid sequence of an immunoregulatory peptide from calf thymus". Biochemistry. 27(11): 4066-71.
16. Burstein Y, Trainin N. (1999). "THF-gamma2 analogs and pharmaceutical compositions comprising them". US5968898(A)
17. Cefai D, Schwaninger R, Balli M, Brunner T, Gimmi C. (2001). "Functional characterization of Fas ligand on tumor cells escaping active specific immunotherapy." Cell Death Differ 8(7): 687-695.
18. Chandrasekher YA, Mckernan PA. (2010). Method for treatin cervical cancer. JP2010013467(A).
19. Christen U, Von Herrath MG. (2004). "IP-10 and type 1 diabetes: a question of time and location." Autoimmunity 37(5): 273-82.
20. Coulie PG, Van den Eynde BJ, van der Bruggen P, Van Pel A, Boon T. (1997). "Antigens recognized by T-lymphocytes on human tumours." Biochem Soc Trans 25(2): 544-8.
21. Desai D, Brightling C. (2009). "Cytokine and anti-cytokine therapy in asthma: ready for the clinic?" Clin Exp Immunol 158(1): 10-9.
22. Dworacki G, Meidenbauer N, Kuss I, Hoffmann TK, Gooding W, Lotze M, Whiteside TL. (2001) "Decreased zeta chain expression and apoptosis in CD3+ peripheral blood T lymphocytes of patients with melanoma". Clin Cancer Res. 7(3 Suppl):947s-957s.
23. Eklund CM (2009). "Proinflammatory cytokines in CRP baseline regulation." Adv. Clin. Chem 48: 111-36.
24. Gastinel L. N. et. al. (1984) "Studies on the zinc binding site to the serum thymic factor" Biochimica et Biophysica Acta 797(2): 147-155.
25. Ginn JD, Sorcek RJ, Turner MR, Young E, (2007). Substituted 3-amino-thieno(2,3-B) pyridine-2-carboxamide compounds, their preparation and use. WO2007146602A1
26. Grange JM, Bottasso O, Stanford CA, Stanford JL. (2008). "The use of mycobacterial adjuvantbased agents for immunotherapy of cancer." Vaccine 26(39): 4984-90.
27. Granoth, R. et. al. (1997) "Tuftsin-THF-γ2 chimeric peptides: potential novel immunomodulators" Immunopharmacology. 37(1):43-52
28. Haddad, J. (2009) "Thymulin and zinc (Zn2+)-mediated inhibition of endotoxin-induced production of proinflammatory cytokines and NF-_B nuclear translocation and activation in the alveolar epithelium: Unraveling the molecular immunomodulatory, anti-inflammatory effect of thymulin/Zn2+ in vitro". Molecular Immunology 47: 205-214.
29. Hadeen JW. (2009) "Vaccine immunotherapy for immune supressed patients". US20090180982A1.
30. Handzel ZT, B. Y., Buchner V, Pecht M, Trainin N. (1990). "Immunomodulation of T cell deficiency in humans by thymic humoral factor: from crude extract to synthetic thymic humoral factor-gamma 2." J Biol Response Mod. 9(3): 269-78.
31. Hanje AJ, Chalasani N. (2007). "How common is chronic liver disease from acute druginduced liver injury?" Gastroenterology 132: 2067-8.
32. Holtan SG, Creedon DJ, Haluska P, Markovic SN. (2009). "Cancer and pregnancy: parallels in growth, invasion, and immune modulation and implications for cancer therapeutic agents." Mayo Clin Proc 84(11): 985-1000.
33. Hsu HH. (2008). Method for treating hepatitis C virus infection in treament failure patients. US20080213218A1.
34. Isoda S, Aibara S, Miwa T, Fujiwara H, Yokohama S, Matumoto H. (1989) Process for preparing tricyclic triazolopyrimidinic derivatives. YU22088(A)
35. Janeway C, T. P., Walport M, Shlomchik M. (2001). Immunobiology. New York and London, Garland Publishing.
36. Jassoy C, Bussmann BM, Reiche S. (2010). Method and means for activating memory B lymphocytes. WO2010023218 (A1).
37. Jiménez SA, Derk CT. (2004). "Following the molecular pathways toward an understanding of the pathogenesis of systemic sclerosis." Ann Intern Med 140(1): 37-50.
38. Kaushansky K. (2005) "The molecular mechanisms that control thrombopoiesis". J Clin Invest. 115(12):3339-47
39. Kaushansky K. (2009). "Determinants of platelet number and regulation of thrombopoiesis." Hematology Am Soc Hematol Educ Program. 2009: 147-52.
40. Kook Al, Yakir Y, Trainin N. (1975). Isolation and partial chemical characterization of THF, a thymus hormone involved in immune maturation of lymphoid cells. Cell Immunol., 19: 151-157.
41. Kreitman RJ (2009). "Recombinant immunotoxins containing truncated bacterial toxins for the treatment of hematologic malignancies." BioDrugs 23(1):1-13.
42. Luqman M, Klabunde S, Lin K, Georgakis GV, Cherukuri A, Holash J, Goldbeck C, Xu X, Kadel EE 3rd, Lee SH, Aukerman SL, Jallal B, Aziz N, Weng WK, Wierda W, O'Brien S, Younes A. (2008) "The antileukemia activity of a human anti-CD40 antagonist antibody, HCD122, on human chronic lymphocytic leukemia cells". Blood. 112(3):711-20.
43. MacLean GD, Miles DW, Rubens RD, Reddish MA, Longenecker BM. (1996). "Enhancing the effect of theratope STn-KLH cancer vaccine in patients with metastatic breast cancer by pretreatment with low-dose intravenous cyclophosphamide." J Immunother Emphasis tumor Immunol 19(4): 309-316.
44. Maher J, Davies E. (2004). "Targeting cytotoxic T lymphocytes for cancer immunotherapy." British Journal of Cancer 91: 817-821.
45. Mallat A, Fouassier L, Préaux AM, Gal CS, Raufaste D, Rosenbaum J, Dhumeaux D, Jouneaux C, Mavier P, Lotersztajn S. (1995) "Growth inhibitory properties of endothelin-1 in human hepatic myofibroblastic Ito cells. An endothelin B receptor-mediated pathway". J Clin Invest. 96(1):42-9.
46. Mastrangelo MF, Maguire HC Jr, Sato T, Nathan FE, Berd D. (1996). "Active specific immunization in the treatment of patients with melanoma." Seminars in Oncology 23(6): 773-781.
47. Mathew M, Verma RS. (2009). "Humanized immunotoxins: a new generation of immunotoxins for targeted cancer therapy." Cancer Sci 100(8): 1359-65.
48. McDermott DF. (2009) "Immunotherapy of metastatic renal cell carcinoma". Cancer. 115(10 Suppl):2298-305.
49. Michelsen KS, Wong MH, Shah PK, Zhang W, Yano J, Doherty TM, Akira S, Rajavashisth TB, Arditi M. (2004). "Lack of Toll-like receptor 4 or myeloid differentiation factor 88 reduces atherosclerosis and alters plaque phenotype in mice deficient in apolipoprotein E." Proc Natl Acad Sci U S A 101 (29): 10679-84.
50. Miossec P, Ling T. (2010). Drug for treating gastric cancer. US2010055108 (A1).
51. Misaouel P, Dispenzieri A, Galanis E. (2009). "Clinical testing of engineered oncolytic measles virus strains in the treatment of cancer: an overview." Curr Opin Mol Ther 11(1): 43-53.
52. Mocchegiani E., Muzzioli M., Cipriano C., Giacconi R. (1998) "Zinc, T-cell pathways, aging: Role of metallothioneins". Mech Ageing Dev. 106 (1-2):183-204
53. Nishimoto N, Kishimoto , Adachi Y, Takayama K. (2010). "Therapeutic agent for mesothelioma comprising interleukin-6". NZ546557 (A).
54. Novobrant-Seva T, Violette S, Koteliansky V, Ibraghimov A. (2006). Methods for treating fibrotic conditions. WO2006125140 (A2)
55. Ophir R, P. M., Burstein Y, Harshemesh H, Ben-Efraim S, Trainin N. (1991). "Therapeutic effectiveness against MOPC-315 plasmacytoma of low or high doses of the synthetic thymic hormone THF-gamma 2 in combination with an "immunomodulating" or a "non-immunomodulating" drug." Int J Cancer. 48(1): 96-100.
56. Oude Nijhuis MM, van Keulen JK, Pasterkamp G, Quax PH, de Kleijn DP. (2007). "Activation of the innate immune system in atherosclerotic disease." Curr Pharm Des. 13(10): 983-94.
57. Pasqualini D. (1996). "A retrospective view of tumor immunology." Medicina 56(1): 3-12.
58. Pasterkamp G, Van Keulen JK, De Kleijn DP. (2004). "Role of Toll-like receptor 4 in the initiation and progression of atherosclerotic disease." Eur J Clin Invest 34(5): 328-34.
59. Pawelec G. (2004). " Immunotherapy and immunoselection - tumor escape as the final hurdle." FEBS Letters 567: 63-66.
60. Pecht M, Lourie S., Burstein Y, Zipori D, Trainin N. (1993). "Potentiation of myeloid colony-formation in bone bone marrow of intact and thymectomized mice by the thymic hormone THF-gamma 2." Exp Hematol. 21(2): 277-82.
61. Perez-Diez A, Joncker NT, Choi K, Chan WF, Anderson CC, Lantz O, Matzinger P. (2007). "CD4 cells can be more efficient at tumor rejection than CD8 cells." Blood 109(12): 5346-54.
62. Rager-Zisman, B.; Segev, Y.; Blagerman, S.; Palmon, A.; Tel-Or, S.; Pecht, M.; Trainin, N.; Burstein, Y. (1993) "Thymic humoral factor , THF -_ 2 , enhances immunotherapy of murine cytomegalovirus (MCMV) infection by both CD4 + and CD8 + immune T cells". Immunology Letters. 39(1):23-31.
63. Sahin U, Türeci O, Pfreundschuh M. (1997). "Serological identification of human tumor antigens." Curr Opin Immunol 9(5): 709-16.
64. Sanda MG, Smith DC, Charles LG, Hwang C, Pienta KJ, Schlom J, Milenic D, Panicali D, Montie JE. (1999). "Recombinant vaccinia-PSA (PROSTVAC) can induce a prostate-specific immune response in androgen-modulated human prostate cancer." Urology 53(2): 260-6.
65. Shaker MA, Younes HM. (2009) "Interleukin-2: evaluation of routes of administration and current delivery systems in cancer therapy". J Pharm Sci. 98(7):2268-98.
66. Shumway NM, Ibrahim N, Ponniah S, Peoples GE, Murray JL. (2009). "Therapeutic breast cancer vaccines: a new strategy for early-stage disease." BioDrugs 23(5): 277-87.
67. Sicard H. (2010). "Improved methods of using phosphoantigens toghether with interleukin-2 for the treatment of cancer". EP2150258 (A2).
68. Sitkovsky M. (2007). "Modulation of immune response and inflamation by targeting hypoxia inducible factors". US20070249550A1.
69. Smith III GJ, WILLS KN (2009) "Antigenic tau peptides and uses thereof". US2011177109 (A1)
70. Soddu S, Lewis A. (1992). "Driving adenovirus type 12-transformed BALB/c mouse cells to express high levels of class I major histocompatibility complex proteins enhances, rather than abrogates, their tumorigenicity." J. Virol. 66(5): 2875-2884.
71. Teschke R, Schwarzenboeck A, Hennermann KH. (2008). " Kava hepatotoxicity: a clinical survey and critical analysis of 26 suspected cases." Eur J Gastroenterol Hepatol 20: 1182-93.
72. Tillmann HL, McHutchison JG. (2010). "Use of thrombopoietic agents for the thrombocytopenia of liver disease." Semin Hematol. 47(3): 266-73.
73. Tran H, Nourse J, Hall S, Green M, Griffiths L, Gandhi MK. (2008). "Immunodeficiency-associated lymphomas." Blood Rev 22(5): 261-81.
74. Trainin N, Burstein Y. (1986) Novel THF compositions. EP0204328(A2)
75. Trimble CL, Frazer IH. (2009). "Development of therapeutic HPV vaccines." Lancet Oncol 10(10): 975-80.
76. Van den Eynde BJ, Boon T. (1997). "Tumor antigens recognized by T lymphocytes." Int J Clin Lab Res 27(2): 81-6.
77. Vaux D (1993). "Toward an understanding of the molecular mechanisms of physiological cell death." Proc. Natl. Acad. Sci. USA 90(3): 786-789.
78. Veber DF (1978) Strachan RG. "Process for the preparation of pyroglutamyl-Ala-Lys-Ser-Gln-Gly-Gly-Ser-Asn". US 4098777 (A)
79. Wang RF. (1999). "Human tumor antigens: implications for cancer vaccine development." J Mol Med 77(9): 640-55.
80. Watkins PB, Seeff LB. (2006). "Drug-induced liver injury: summary of a single topic clinical research conference." Hepatology 43: 618-31.
81. Weber J (2000). "Melanoma peptide vaccines: from preclinical background to clinical trials." Curr Oncol Rep 2(1): 38-47.
82. White B. (2010). "Method of treating systemic lupus erythematosus". US20100143373A1
83. Williams R. (1996) "Classification, etiology and considerations of outcome in acute liver failure". Semin Liver Dis 16(4):343-8
84. Williams R. (2003). "Changing clinical patterns in acute liver failure." J Hepatol 39: 660-1.
85. Witters P, Freson K, Verslype C, Peerlinck K, Hoylaerts M, Nevens F, Van Geet C, Cassiman D. (2008). "Review article: blood platelet number and function in chronic liver disease and cirrhosis." Aliment Pharmacol Ther 27(11): 1017-29.
86. Yang L, Carbone DP. (2004). "Tumor-host immune interactions and dendritic cell dysfunction." Adv Cancer Res 92: 13-27.
87. Zimmerman DH. (2009). Methods of preparation and composition of peptide constructs useful for treatment of rheumatoid arthritis. US. WO2009114869A2.
88. Zou W, Chen L. (2008) "Inhibitory B7-family molecules in the tumour microenvironment". Nat Rev Immunol. 8(6):467-77.

## Claims

1. An immunomodulator metallopeptide, **characterized in that** comprises:
a) A peptide of formula: : Xn-Asp-Gly-Pro-Lys-Phe-Leu-Xm,
Where: Xn and Xm are any independent aminoacid one from another,
n has a value of 0 to 4,
m has a value of 0 to 2, and
b) a divalent ion selecting from the group comprising: Zn2+, Cu2+, Mg2+ o or mixtures thereof, wherein the peptide and the divalent ion are joined between each other by a noncovalent linking.

2. The immunomodulator metallopeptide according to claim 1, **characterized in that** the divalent ion is Zn2+.

3. The immunomodulator metallopeptide according to claim 1, **characterized in that** the peptide is selected from the group comprising: Leu-Glu-Asp-Gly-Pro-Lys-Phe-Leu (SEQ. ID. No. 1), Glu-Asp-Gly-Pro-Lys-Phe-Leu (SEQ. ID. No. 2), Asp-Gly-Pro-Lys-Phe-Leu (SEQ. ID. No. 3), X-Leu-Glu-Asp-Gly-Pro-Lys-Phe-Leu (SEQ. ID. No. 4), X-X-Leu-Glu-Asp-Gly-Pro-Lys-Phe-Leu (SEQ. ID. No. 5), Leu-Glu-Asp-Gly-Pro-Lys-Phe-Leu-X (SEQ. ID. No. 6), Leu-Glu-Asp-Gly-Pro-Lys-Phe-Leu-X-X (SEQ. ID. No. 7) y X-Leu-Glu-Asp-Gly-Pro-Lys-Phe-Leu-X (SEQ. ID. No. 8).

4. The immunomodulator metallopeptide according to claim 3, **characterized in that** the divalent ion is Zn2+.

5. The immunomodulator metallopeptide according to claim 4, **characterized in that** the peptide has the formula: Leu-Glu-Asp-Gly-Pro-Lys-Phe-Leu (SEQ. ID. No. 1).

6. The immunomodulator metallopeptide according to any of claims 1 to 5, **characterized in that** the p/p ratio of zinc, magnesium or cooper: peptide of formula I is of 1 : 0.1 a 1 : 10,000.

7. A pharmaceutical composition with immunomodulator effect, **characterized in that** comprises the immunomodulator metallopeptide according to claims 1 to 6, and a vehicle pharmaceutically acceptable.

8. The pharmaceutical composition according to claim 7, **characterized in that** the immunomodulator metallopeptide has a p/p concentration of 0.00001% a 99.999%.

9. A nutraceutical composition with immunomodulator effect, **characterized in that** comprises the immunomodulator metallopeptide according to claims 1 to 6, and a vehicle nutraceuticaly acceptable.

10. The nutraceutical composition according to claim 9, **characterized in that** the immunomodulator metallopeptide has a p/p concentration of 0.00001% a 99.999%.

11. The immunomodulator metallopeptide according to claims 1 to 6, to be used as antiinflammatory agent or as immunomodulator agent or immunostimulating in humans and animals, in therapeutically effective amounts and posology.

12. The immunomodulator metallopeptide according to claims 1 to 6, to be used in for increasing the quantity of T lymphocytes in the blood, both cooperatives (CD4+) as regulators (CD4+ CD25+) in humans and animals, in therapeutically effective amounts and posology.

13. The immunomodulator metallopeptide according to claims 1 to 6, to be used in the infectious diseases treatment, either acute, chronic or recurrent, to treat fibrosis secondary to a chronic inflammation, treating hepatitis, cirrhosis, cancer and metastasis, plaquetopenia, or thrombocytopenia, and for the treatment of autoimmune diseases or immunologic hypersensitivity, in human and animals, in therapeutically effective amounts and posology.

14. The immunomodulator metallopeptide according to claims 1 to 6, to be used in the treatment of any stress condition meaning any immunosuppression degree in the human or animal organism, in therapeutically effective amounts and posology.

15. The immunomodulator metallopeptide according to claims 1 to 6, to be used in for improving the response to the organism, human or animal, to the vaccines.

16. The use of the immunomodulator metallopeptide of claims 1 to 6, to prepare an antiinflammatory medicament.

17. The use of the immunomodulator metallopeptide of claims 1 to 6, to prepare an immunomodulator or immunostimulating medicament.

18. The use of the immunomodulator metallopeptide of claims 1 to 6, to prepare a medicament or nutraceutical for increasing the quantity of T lymphocytes in the blood, both cooperatives (CD4+) as regulators (CD4+ CD25+) in humans and animals.

19. The use of the immunomodulator metallopeptide of claims 1 to 6, to prepare a medicament or nutraceutical for treating infectious diseases either acute, chronic or recurrent, to treat fibrosis secondary to a chronic inflammation, treating hepatitis, cirrhosis, cancer and metastasis, plaquetopenia, or thrombocytopenia, and for the treatment of autoimmune diseases or immunologic hypersensitivity.

20. The use of the immunomodulator metallopeptide of claims 1 to 6, to prepare a medicament or nutraceutical for treating any stress condition meaning any immunosuppression degree.

21. The use of the immunomodulator metallopeptide of claims 1 to 6, to prepare a medicament or nutraceutical for improving the response of the organism to vaccines or infections.

22. The use of the immunomodulator metallopeptide of claims 1 to 6, wherein the immunomodulator metallopeptide is administered in dosage of about 2 to 2000 ng/Kg of body weight, distributed in two or more intakes, administered with an interval of 1 to 10 days between one and another, depending of the animal species, age, health, or genre.

23. A medicament or nutraceutical, **characterized by** containing as drug or active ingredient the immunomodulator metallopeptide according to any of claims 1 to 6.

24. A method for obtaining a immunomodulator metallopeptide, **characterized in that** comprises the steps of:
a) mixing the peptide of formula: : Xn-Asp-Gly-Pro-Lys-Phe-Leu-Xm,
Where: Xn and Xm are any independent aminoacid one from another,
n has a value of 0 to 4,
m has a value of 0 to 2, with a buffer solution at Ph 7 to 7.5,
b) adding to the mixture obtained in a) a solution containing a divalent ion selected from the group comprising Zn2+, Cu2+, Mg2+ o and mixtures thereof, and
c) resting the mixture obtained in b) at 4°C in darkness during the enough time until the metallopeptide formation.

25. The method according to claim 24, **characterized in that** the divalent ion is Zn2+.

26. The method according to claim 24, **characterized in that** the peptide is selected from the group comprising: Leu-Glu-Asp-Gly-Pro-Lys-Phe-Leu (SEQ. ID. No. 1), Glu-Asp-Gly-Pro-Lys-Phe-Leu (SEQ. ID. No. 2), Asp-Gly-Pro-Lys-Phe-Leu (SEQ. ID. No. 3), X-Leu-Glu-Asp-Gly-Pro-Lys-Phe-Leu (SEQ. ID. No. 4), X-X- Leu-Glu-Asp-Gly-Pro-Lys-Phe-Leu (SEQ. ID. No. 5), Leu-Glu-Asp-Gly-Pro-Lys-Phe-Leu-X (SEQ. ID. No. 6), Leu-Glu-Asp-Gly-Pro-Lys-Phe-Leu-X-X (SEQ. ID. No. 7) y X-Leu-Glu-Asp-Gly-Pro-Lys-Phe-Leu-X (SEQ. ID. No. 8).

27. The method according to claim 26, **characterized in that** the divalent ion is Zn2+.

28. The method according to claim 27, **characterized in that** the peptide has the formula: Leu-Glu-Asp-Gly-Pro-Lys-Phe-Leu (SEQ. ID. No. 1).

29. The method according to any of claims 24 to 28, **characterized in that** the p/p ratio of zinc : peptide of formula I is of 1 : 0.1 a 1 : 10,000.
